# FASCICULE DE BREVET EUROPEEN

(11) **EP 1 542 729 B1**
(45) Date de publication et mention de la délivrance du brevet: **06.11.2013**
(21) Numéro de dépôt: 03772373.1
(22) Date de dépôt: 16.09.2003
(51) Int. Cl.: A61K 47/48, A61K 39/385

(54) **PRODUIT IMMUNOGENE STABLE COMPRENANT DES HETEROCOMPLEXES ANTIGENIQUES**
STABILES IMMUNOGEN PRODUKT ENTHALTEND ANTIGENISCHE HETERO-KOMPLEXE
STABLE IMMUNOGENIC PRODUCT COMPRISING ANTIGENIC HETEROCOMPLEXES

(30) Priorité: 16.09.2002 FR 0211455
(43) Date de publication de la demande: 22.06.2005
(73) Titulaire: NEOVACS, 75116 Paris (FR)
(72) Inventeur: LE BUANEC, Hélène, F-75005 PARIS (FR); ZAGURY, Daniel, F-75007 PARIS (FR)
(74) Mandataire: Jupin, Claude Christian Marie
(86) Numéro de dépôt international: PCT/FR2003/002733
(87) Numéro de publication internationale: WO 2004/024189

(56) Documents cités:
- WO-A-00/03732
- WO-A-02/11759
- WO-A-02/22164
- WO-A-91/01146
- WO-A-96/27389
- WO-A-99/57981
- FR-A1- 2 759 296
- US-A- 6 093 405
- US-B1- 6 340 461
- DATABASE CA [Online] CHEMICAL ABSTRACTS SERVICE, COLUMBUS, OHIO, US; SHIRAKAWA, TAKASHI ET AL: "VEGF121-specific monoclonal antibodies, hybridomas producing them, and determination of VEGF using the antibodies" XP002242469 accession no. STN Database accession no. 131:70855 & JP 11 178593 A (FUJIREBIO, INC., JAPAN) 6 juillet 1999 (1999-07-06)
- KIM S K ET AL: "Comparison of the effect of different immunological adjuvants on the antibody and T-cell response to immunization with MUC1-KLH and GD3-KLH conjugate cancer vaccines." VACCINE. ENGLAND 2000, vol. 18, no. 7-8, 2000, pages 597-603, XP002242468 ISSN: 0264-410X
- RICHARD M ET AL: "ANTI-IL-9 VACCINATION PREVENTS WORM EXPULSION AND BLOOD EOSINOPHILIA IN TRICHURIS MURIS-INFECTED MICE", PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.97.2.767, vol. 97, no. 2, 18 January 2000 (2000-01-18), pages 767-772, XP000985925, ISSN: 0027-8424
- SCOTT ET AL: "When is a search not a search? The EPO approach", WORLD PATENT INFORMATION, ELSEVIER, AMSTERDAM, NL, vol. 29, no. 2, 5 April 2007 (2007-04-05), pages 108-116, XP022021330, ISSN: 0172-2190, DOI: 10.1016/J.WPI.2006.10.011

## Description

### DOMAINE DE L'INVENTION

La présente invention décrit à des produits immunogènes stables comprenant des hétérocomplexes protéiques immunogènes pour l'obtention d'une réponse immunitaire humorale avec production d'anticorps spécifiques dirigés à l'encontre d'un ou plusieurs antigènes, en particulier à l'encontre d'un antigène du « soi », ainsi qu'à leur utilisation dans le domaine des vaccins.

### ART ANTERIEUR

L'obtention d'une réponse anticorps de haut niveau à l'encontre d'un antigène donné, chez un individu, est un objectif couramment recherché, que l'antigène soit un antigène « étranger » ou un antigène du « soi ».

Toutefois, le problème d'une bonne reconnaissance de l'antigène à l'encontre duquel une réponse anticorps est recherchée, chez un individu, doit être résolu dans un certain nombre de cas, notamment (a) lorsque l'antigène d'intérêt se comporte comme un « haptène », c'est à dire une structure chimique de faible masse moléculaire qui est peu ou pas immunogénique sous forme libre, mais qui, une fois fixée sur une molécule de haut masse moléculaire, est capable d'induire la production d'anticorps spécifiques de cet haptène, et (b) lorsque l'antigène d'intérêt est une protéine du « soi », c'est à dire une protéine qui est produite naturellement chez l'individu, pour laquelle il existe une tolérance immunitaire due à la délétion des clones de lymphocytes T correspondants, au cours du développement du système immunitaire..

Afin de provoquer, ou d'augmenter, la reconnaissance d'un antigène d'intérêt par les cellules B, on a réalisé diverses constructions immunogènes dans l'état de la technique.

Une première forme de ces constructions immunogènes consiste en un couplage covalent de l'antigène d'intérêt sur une molécule porteuse, la molécule porteuse apportant des structures reconnues par les lymphocytes T auxiliaires (cellules « T helper »), en association avec des molécules de classe II du Complexe Majeur d'Histocompatibilité (CMH), et qui activent les lymphocytes T auxiliaires qui produisent alors diverses cytokines, dont l'IL-2, lesquelles cytokines vont à leur tour activer les clones de cellules 8 spécifiques de l'antigène d'intérêt. Les cellules B spécifiques de l'antigène d'intérêt, une fois activées, vont se multiplier et produire des anticorps spécifiques de l'antigène d'intérêt, ce qui est l'objectif recherché. En général, ce type de constructions immunogènes consiste en des produits du coulage chimique covalent entre l'antigène d'intérêt et la molécule porteuse, lesquels, après une étape de purification et élimination des produits non couplés, sont des produits finaux de structure chimique bien définie.

La première forme d'une construction immunogène ci-dessus est par exemple illustrée par l'article de Richard et al. qui décrit la préparation de produits du couplage covalent entre l'IL-9 et l'ovalbumine (Proc. Natl. Acad. Sci. USA, Vol. 97(2) : 767-772). Elle est également illustrée dans le brevet américain n° US 6,340,461 (Terman), qui décrit des produits de couplage entre une ou plusieurs copies d'un antigène d'intérêt, à l'encontre duquel une réponse anticorps spécifique est recherchée chez un individu, et une molécule porteuse constituée d'un « Superantigène ». L'antigène d'intérêt est couplé de manière exclusivement covalente à la molécule porteuse, par exemple à l'aide du glutaraldéhyde (aussi appelé « pentanedial »), les produits non couplés de manière covalente étant éliminés afin d'obtenir un produit final chimiquement bien défini.

D'autres produits de couplage covalents ont été décrits dans l'état de la technique. L'article de Kim et al. (1999, Vaccine, Vol. 17 (n° 7-8) : 597-603) décrit des immuno-conjugués comprenant un antigène tumoral choisi parmi MUC1 et GD3 qui est couplé à la protéine porteuse KLH. Kim et al. (1999) ont décrit le rôle des immuno-adjuvants dans l'induction d'une réponse anticorps et d'une réponse cellulaire de type T à l'encontre de ces immuno-conjugués. La demande PCT n° WO 96/27389 décrit des composés immunogènes dénués de toxicité dérivant d'une protéine de régulation d'un virus VIH-1, VIH-2 ou HTLV-2, par traitement chimique à l'aide d'un agent de couplage tel qu'un aldéhyde, ou d'un protéine porteuse activée par un pré-traitement à l'aide d'un aldéhyde. La demande PCT n° WO 02/11759 décrit des vaccins renfermant à titre de principe actif un immunogène qui est un facteur cytokinique ou un facteur de régulation cellulaire à propriétés immunosuppressives/angiogéniques. En particulier, la demande WO 02/11759 décrit des vaccins comprenant des immuno-conjugués entre diverses cytokines et une protéine porteuse.

Eventuellement, le produit du couplage covalent entre l'antigène d'intérêt et le superantigène peut être préparé sous forme d'un polymère dudit produit de couplage, par exemple par fixation non covalente des produits de couplage monomères entre eux, au travers d'interactions ioniques, d'interactions d'adsorption ou encore d'interactions biospécifiques. Par exemple, les produits de couplage monomères peuvent former des complexes avec des molécules hautement chargées positivement ou négativement, grâce à des ponts salins réalisés dans des conditions de faible force ionique. De grands complexes de produits de couplage monomères sont préparés en utilisant des polymères chargés, tels que des polymères poly(acide L-glutamique) ou poly(L-lysine). Selon un autre mode de réalisation d'un polymère de produits de couplage monomères, les produits du couplage exclusivement covalent entre l'antigène d'intérêt et le superantigène peuvent être adsorbés ou couplés de manière non covalente à la surface de microparticules, telles que des billes de latex ou d'autres polymères hydrophobes.

Une seconde forme de ces constructions immunogènes, désignée communément structure « MAP » (pour Multi-Antigenic Protein ») se présente en général sous la forme d'un squelette protéique constitué d'un polymère de poly(lysine), linéaire ou branché, sur lequel sont fixés de manière covalente un ou plusieurs antigènes d'intérêt.

Une troisième forme de ces constructions immunogènes consiste en des microparticules sur lesquelles sont fixés le ou les antigènes d'intérêt. On connaît des formes variées de microparticules porteuses d'antigènes.

On connaît par exemple les iscomes (pour « immunostimulating complexes »), qui sont constitués d'un complexe antigénique et d'un adjuvant, le composé QuilA.

On connaît aussi les liposomes, qui possèdent les mêmes désavantages que les iscomes, à savoir notamment une certaine toxicité et des effets immunologiques secondaires, dus à leur manque de pureté.

On connaît également les microparticules biodégradables, comme les polymères d'acide lactique et d'acide glutamique (Aguado et Lambert, 1992, Immuno. Biol., Vol. 184: 113-125) ou encore des particules d'amidon (Demande de brevet américain n° 2002/0098203 - Gutavsson et al.), dans la matrice polymérique desquelles sont emprisonnés les antigènes d'intérêt. Ces particules libèrent l'antigène sous leur forme soluble pendant la dégradation de la matrice polymérique.

On a aussi décrit des particules constituées exclusivement de protéines recombinantes hybrides, comme décrit dans la demande de brevet français n° FR 2 635 532 (Thiollais et al.).

On connaît également des microsphères poreuses dans lesquels les antigènes sont immobilisés à l'intérieur des micropores par captation ou couplage physique, comme décrit dans le brevet américain n° US 5,008116 (Cahn).

Toutefois, les différentes solutions proposées dans l'état de la technique ont toutes en commun au moins un inconvénient technique lié à leur mode de préparation, à savoir la perte d'une grande proportion du matériel antigénique d'intérêt, du fait d'une étape obligatoire d'éliminatlon des antigènes non couplés ou non adsorbés.

De plus, si les techniques antérieures permettent de réaliser une association entre un antigène d'intérêt de faible masse moléculaire avec une molécule porteuse, elles sont en général inadaptées au couplage d'un antigène d'intérêt de haut masse moléculaire, par exemple de plus de 10 kDa, à la molécule porteuse, en raison notamment des encombrements stériques qui interdisent le couplage d'un nombre élevé de molécules d'antigènes d'intérêt de haut masse moléculaire à une même molécule porteuse.

Enfin, la plupart, sinon la totalité, des constructions antigéniques peptidiques connues englobent dans leur structure une seule molécule porteuse, ce qui est un inconvénient technique lorsque l'objectif est d'induire une réponse immunitaire préventive ou thérapeutique à fois contre l'antigène d'intérêt et la molécule porteuse elle-même.

Il existe donc un besoin dans l'état de la technique pour des constructions immunogènes améliorées permettant la production d'un haut niveau d'anticorps spécifiques d'un antigène d'intérêt chez un individu chez lequel une telle réponse immunitaire humorale est recherchée, qui soient peu coûteuses, simples à préparer et qui puissent être synthétisées de manière reproductible.

### SOMMAIRE DE L'INVENTION

La présente invention décrit de nouvelles constructions immunogènes permettant de résoudre les divers problèmes techniques rencontrés avec les constructions Immunogènes connues dans l'art antérieur et qui permettent de combler les différents besoins techniques décrits ci-dessus.

L'invention a pour objet un produit immunogène stable pour l'induction d'anticorps à l'encontre d'une ou plusieurs protéines antigéniques chez un sujet, caractérisé en ce qu'il consiste en des hétérocomplexes immunogènes protéiques constitués d'associations entre (i) des molécules de protéines antigéniques et (ii) des molécules protéiques porteuses et en ce que au moins 1 pour cent et en ce que moins de 40 pour cent des protéines antigéniques (i) sont liées avec les molécules protéiques porteuses (ii) par une liaison covalente, et en ce que la ou les protéines antigéniques (i) consistent en des cytokines produites naturellement par ledit sujet.

L'invention décrit aussi un produit immunogène comprenant des hétérocomplexes immunogènes protéiques stables pour l'induction d'anticorps à l'encontre d'une ou plusieurs protéines antigéniques chez un sujet, chaque hétérocomplexe comprenant (i) une pluralité de protéines antigéniques, liées à (ii) une molécule protéique porteuse, caractérisé en ce que, dans ledit produit immunogène, au moins 1 pour cent et en ce que moins de 40 pour cent des protéines antigéniques (i) sont liées aux molécules protéiques porteuses (ii) par une liaison covalente.

De préférerice, l'hétérocomplexe immunogène constitutif dudit produit immunogène comprend de 5 à 50 protéines antigéniques (i) pour une molécule protéique porteuse (ii), de préférence de 20 à 40 protéines antigéniques (i) pour une molécule protéique porteuse (ii).

De préférence, les liaisons covalentes entre une ou plusieurs des protéines antigéniques (i) et les molécules protéiques porteuses (ii) sont réalisées par l'intermédiaire d'un agent chimique de liaison fonctionnelle.

Par molécule d'intérêt antigénique, on entend toute protéine comprenant un ou plusieurs épitopes B de la d'une protéine antigénique native à l'encontre de laquelle la production d'anticorps est recherchée. Ladite molécule d'intérêt antigénique peut consister en la protéine native elle-même ou un dérivé protéique de la protéine native, tels qu'un fragment peptidique de la protéine native, ou encore toute forme biologiquement inactivée de la protéine native obtenue par traitement chimique, physique ou par mutation génétique. La protéine d'intérêt antigénique peut aussi consister en un homo-oligomère ou homo-polymère de la protéine native ou encore en un homo-oligomère ou homo-polymère d'un fragment peptidique de la protéine native. La protéine d'intérêt antigénique peut aussi peut aussi consister en un hétéro-oligomère ou en un hétéro-polymère comprenant une combinaison de plusieurs fragments peptidiques distincts initialement inclus dans la protéine native..

Selon le mode de réalisation général d'un produit immunogène tel que décrit, la molécule protéique porteuse (ii) est une protéine immunogène induisant la production de lymphocytes T helper et/ou de lymphocytes T cytotoxiques dirigés à l'encontre de cellules présentant à leur surface ladite molécule protéique porteuse, ou tout peptide qui en est dérivé, en association avec des molécules présentoirs du Complexe Majeur d'Histocompatibilité (MHC), respectivement de classe II et/ou de classe I. La molécule protéique porteuse (ii) peut être également une protéine immunogène induisant à la fois la production de lymphocytes T helper et la production d'anticorps par des lymphocytes B dirigés contre la protéine porteuse.

Selon un mode de réalisation particulier d'intérêt, le produit immunogène est caractérisé en ce que la molécule protéique porteuse (ii) est une protéine immunogène induisant la production de lymphocytes cytotoxiques dirigés à l'encontre de cellules présentant à leur surface ladite molécule protéique porteuse, ou tout peptide qui en est dérivé, en association avec des molécules de classe I du Complexe Majeur d'Histocompatibilité (MHC).

Les produits immunogènes préférés sont choisis parmi les produits immunogènes comprenant les hétérocomplexes suivants, dans lesquels les protéines antigéniques (i), d'une part et la molécule porteuse protéique (ii), d'autre part, sont respectivement :
a) (i) IL-4 et (ii) KLH ;
b) (i) interféron alpha et (ii) KLH ;
c) (i) VEGF et (ii) KLH ;
d) (i) IL-10 et (ii) KLH ;
e) (i) interféron alpha et (ii) gp160 de VIH1
f) (i) IL-4 et (ii) l'antigène allergène Bet v 1 ; et
g) (i) le VEGF et (ii) la protéine E7 d'un Papillomavirus.
h) (i) la protéine Tat de VIH1 biologiquement inactivée et (ii) la protéine gp 120 de VIH1.
i) (i) un anticorps humain d'isotype IgE et (ii) la protéine Tat de VIH1 inactivée ;
j) (i) le fragment β de la ricine et (ii) KLH.

L'invention décrit également une composition, notamment une composition pharmaceutique, une composition immunogène ou une composition de vaccin, caractérisée en ce qu'elle comprend un produit immunogène tel que défini ci-dessus.

Elle a également trait à un procédé de préparation d'un produit immunogène selon l'une des revendications 1 à 16, caractérisé en ce qu'il comprend les étapes suivantes : .
a) incuber les protéines antigéniques (i) consistant en des cytokines produites naturellement par un sujet, et la molécule porteuse (ii) dans un rapport molaire (i) : (ii) de 5:1 à 50:1, en présence d'un agent chimique de liaison;
b) stabiliser les hétérocomplexes formés à l'étape a) par traitement avec le formaldéhyde ;
c) récupérer le produit immunogène comprenant les hétérocomplexes immunogènes qui est préparé à l'étape b).

### DESCRIPTION DES FIGURES

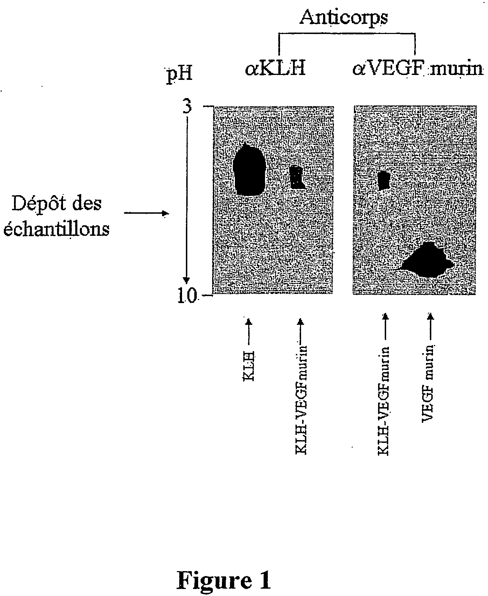

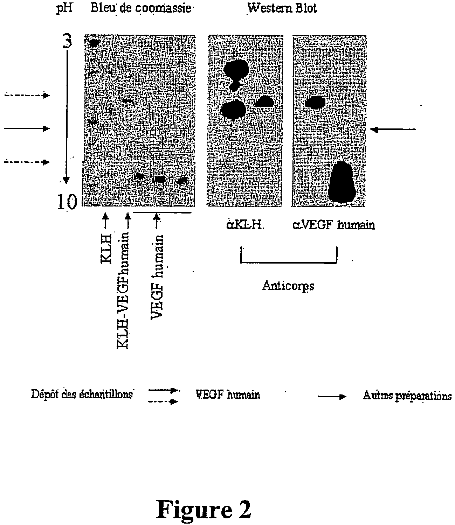

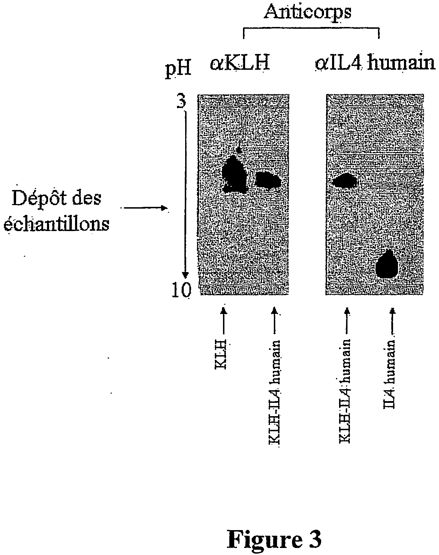

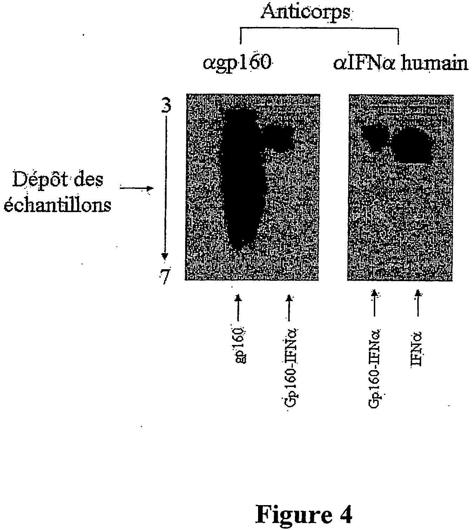

**La** **Figure 1** illustre la caractérisation du produit immunogène comprenant des hétérocomplexes KLH-VEGF murin par isoélectrofocalisation en gel d'agarose suivie d'une révélation des protéines par immuno-empreinte (« Western Blot »).
**La** **Figure 2** illustre la caractérisation du produit immunogène comprenant des hétérocomplexes KLH-VEGF humain par isoélectrofocalisation avec révélation au Bleu de coomassie, suivie d'une immuno-empreinte (« Western Blot »). A gauche de la Figure est représenté le gel d'isoélectrofocalisation. A droite de la Figure sont représentés les gels d'immuno-empreinte en utilisant des anticorps anti-KLH (à gauche) ou anti-VEGF humain (à droite).
**La** **Figure 3** illustre la caractérisation caractérisation du produit immunogène comprenant des hétérocomplexes KLH-IL4 humain par isoélectrofocalisation en gel d'agarose suivie d'une révélation des protéines par immuno-empreinte (« Western Blot »).
**La** **Figure 4** illustre la caractérisation du produit immunogène comprenant des hétérocomplexes gp 160-IFNα, par isoélectrofocalisation en gel d'agarose suivie d'une révélation des protéines par immuno-empreinte (« Western Blot »).
La **Figure 5** illustre l'activité immunogénique (humorale) du produit immunogène KLH-VEGF murin, par détermination du titre anticorps obtenu après immunisation des souris. **Figure 5A** : souris immunisées avec le VEGF murin. **Figure 5B** : souris immunisées avec le produit immunogène comprenant des hétérocomplexes KLH-VEGF. **Figure 5C** : souris témoins injectées avec l'Adjuvant Incomplet de Freund (AIF).
La **Figure 6** l'activité immunogénique (humorale) du produit immunogène KLH-VEGF murin, par détermination du pouvoir neutralisant des anticorps obtenus après immunisation, vis-à-vis de l'activité angiogénique de la protéine VEGF.
La **Figure 7** illustre l'activité immunogénique (humorale) du produit immunogène KLH-VEGF humain par détermination du titre anticorps obtenu après immunisation des souris.
La **Figure 8** illustre l'activité immunogénique (humorale) du produit immunogène KLH-VEGF humain, par détermination du pouvoir neutralisant des anticorps obtenus après immunisation, vis-à-vis de l'activité angiogénique de la protéine VEGF, mesurée par la prolifération des cellules endothéliales.
La **Figure 9** : illustre l'activité immunogénique (humorale) du produit immunogène KLH-IL4 murin par détermination du titre anticorps obtenu après immunisation.
La **Figure 10** illustre l'activité immunogénique (humorale) du produit immunogène KLH-IL4 murin par détermination du pouvoir neutralisant des anticorps obtenus après immunisation, vis-à-vis de l'activité d'induction de la prolifération des cellules HT-2 par l'IL4.
La **Figure 11** illustre les résultats de production d'anticorps de classe IgG et IgE dirigés contre le Bet v 1, après l'injection de pollen de bouleau, à des souris préalablement immunisées avec un produit immunogène selon l'invention comprenant des hétérocomplexes KLH-IL4.La **Figure 12** illustre l'activité immunogénique (humorale) du produit immunogène KLH-IL4 humain par détermination du pouvoir neutralisant des anticorps obtenus après immunisation, vis-à-vis de l'activité d'induction de la prolifération des cellules TF-1 par l'IL4.

### DESCRIPTION DETAILLEE DE L'INVENTION

L'invention fournit de nouvelles constructions immunogènes induisant un haut niveau de production d'anticorps spécifiques d'un antigène d'intérêt, chez l'individu.

### Les hétérocomplexes protéiques immunogènes de l'invention

On a montré selon l'invention que la production d'un haut niveau d'anticorps spécifiques d'un antigène d'intérêt était obtenu, chez un individu, par l'immunisation de cet individu avec un produit immunogène dans lequel ledit antigène d'intérêt est associé à une molécule protéique porteuse, l'association entre ledit antigène d'intérêt et ladite protéine porteuse étant partiellement covalente et partiellement non covalente.

Plus spécifiquement, on a montré selon l'invention qu'une excellente réponse anticorps à l'encontre d'un antigène d'intérêt est obtenue lorsqu'on immunise un individu avec un produit immunogène stable comprenant des hétérocomplexes protéiques, dans lequel les hétérocomplexes sont constitués d'associations stables entre ledit antigène d'intérêt et ladite molécule protéique porteuse et dans lequel seule une faible proportion de ces associations sont dues à une liaison covalente entre l'antigène d'intérêt et la molécule protéique porteuse, les autres associations entre l'antigène d'intérêt et la molécule protéique porteuse étant réalisées par des liaisons faibles, interactions ioniques, liaisons hydrogènes, forces de Van der Waals, etc.

En particulier, on a montré selon l'invention qu'une réponse anticorps optimale est atteinte lorsque, dans un produit immunogène stable tel que décrit ci-dessus, moins de 40 pour cent des molécules de l'antigène d'intérêt sont liées par une liaison covalente avec les molécules protéiques porteuses. Selon l'invention, une molécule d'antigène d'intérêt liée à une molécule protéique porteuse par « une » liaison covalente signifie que ladite molécule d'antigène d'intérêt est liée de manière covalente, chimiquement, à ladite molécule protéique porteuse, par au moins une liaison covalente, c'est à dire éventuellement par deux liaisons covalentes ou plus.

Le pourcentage de molécules protéiques porteuses et de protéines antigéniques d'intérêt liées entre elles par des liaisons covalentes dans un produit immunogène de l'invention peut être aisément vérifié par l'homme du métier.

Par exemple, la détermination du pourcentage de molécules d'antigène d'intérêt liées aux molécules de protéine porteuse par une liaison covalente dans un produit immunogène de l'invention peut être réalisée selon les étapes suivantes :
(i) soumettre ledit produit immunogène en solution à des conditions dénaturantes et réductrices,
(ii) réaliser une étape de chromatographie d'exclusion de taille avec le produit obtenu à la fin de l'étape (ii) au cours de laquelle les différents constituants protéiques de masse moléculaires décroissants sont élués successivement du support chromatographique d'exclusion de taille.
(iii) mesurer la quantité d'antigène d'intérêt liée par une liaison covalente à la molécule porteuse dans la fraction d'éluat contenant les constituants protéiques ayant le plus haut masse moléculaire ;
(iv) comparer la quantité d'antigène d'intérêt mesurée à l'étape (iii) avec la quantité totale d'antigène d'intérêt inclus initialement dans le produit immunogène de départ.

A l'étape (i) du procédé de détermination du pourcentage de liaisons covalentes décrit ci-dessus, l'incubation d'une quantité donnée (en nombre de moles ou en poids) du produit immunogène de l'invention dans des conditions dénaturantes et réductrices entraîne une dissociation des liaisons faibles entre les différents constituants protéiques non liés entre eux par une liaison covalente.

Des conditions dénaturantes préférées sont la présence d'urée, par exemple à la concentration finale 8M, ou encore la présence de SDS, par exemple à la concentration finale de 1 % en poids total de la solution contenant le produit immunogène. Des conditions réductrices préférées sont la présence de β-mercaptoéthanol, par exemple à la concentration finale de 5% du volume total de la solution contenant le produit immunogène.

A l'étape (ii) du procédé de détermination du pourcentage de molécules d'antigène d'intérêt et de molécules de protéine porteuse liées entre elles par des liaisons covalentes, le support de chromatographie d'exclusion de taille est choisi par l'homme du métier selon ses connaissances générales techniques. Par exemple, l'homme du métier peut avoir recours aux supports chromatographiques commercialisés par la Société Pharmacia sous les noms commerciaux de « Superdex 75^{™} » et de « Superdex 200™ ».

A l'étape (ii), la fraction moléculaire correspondant à la molécule porteuse liée de manière covalente aux molécules d'antigène d'intérêt est éluée en premier lieu, avant la ou les fractions d'éluat contenant l'antigène d'intérêt sous forme libre. L'antigène d'intérêt qui est élué sous forme libre correspond à la fraction d'antigène d'intérêt, qui n'était pas lié par une liaison covalente à la molécule porteuse, au sein du produit immunogène de départ. C'est sur la fraction protéique de haut masse moléculaire qu'est réalisée la mesure de la quantité d'antigène d'intérêt lié de manière covalente à la molécule protéique porteuse, par exemple dans un test immuno- enzymatique, dans un test radioimmunologique ou dans un test par immunofluorescence, direct ou indirect (« sandwich »), en utilisant des anticorps spécifiques de l'antigène d'intérêt et qui ne présentent aucune réaction immunologique croisée avec la molécule protéique porteuse.

A l'étape (iii), on compare la quantité d'antigène d'intérêt lié de manière covalente à la molécule protéique porteuse, qui a été mesurée comme décrit ci-dessus, à la quantité initiale d'antigène d'intérêt incluse initialement dans la quantité donnée (en nombre de moles ou en poids) du produit immunogène de départ, et on calcule ainsi le pourcentage d'antigène d'intérêt qui est lié de manière covalente à la molécule protéique porteuse, dans le produit immunogène de l'invention.

Le pourcentage de molécules protéiques porteuses et de protéines antigéniques d'intérêt liées entre elles par des liaisons covalentes, dans un produit immunogène de l'invention, peut être également aisément vérifiée par l'homme du métier selon une seconde méthode comprenant les étapes suivantes :
a) Immobilisation sur un support d'anticorps dirigés spécifiquement contre la protéine porteuse ;
b) Mise en contact des anticorps contre la protéine porteuse, qui ont été immobilisés sur le support à l'étape a), avec une quantité connue de molécules du produit immunogène à tester comprenant ladite protéine porteuse et une protéine antigénique d'intérêt ;
c) Elimination des molécules du produit immunogène qui ne sont pas liées aux anticorps anti-protéine porteuse immobilisés à l'étape a), à l'aide d'une solution aqueuse tampon comprenant un ou plusieurs agents dénaturant les protéines ;
d)
   d1) Mise en contact (i) des complexes immunogènes formés à l'étape c) entre les anticorps anti-protéine porteuse immobilisés et les molécules du produit immunogène avec (ii) des anticorps dirigés spécifiquement contre la protéine porteuse ;
   d2) Séparément de l'étape d1), mise en contact (i) des complexes immunogènes formés à l'étape c) entre les anticorps anti-protéine porteuse immobilisés et les molécules du produit immunogène avec (ii) des anticorps dirigés spécifiquement contre la protéine antigénique d'intérêt ;
e)
   e1) quantification des anticorps ajoutés à l'étape d1) qui se sont liés à la protéine porteuse ;
   e2) quantification des anticorps ajoutés à l'étape d2) qui se sont liés à la protéine antigénique ;
f) Calcul du rapport entre :
   (i) la quantité d'anticorps fixés anti-protéine porteuse mesurée à l'étape e1) ; et
   (ii) la quantité d'anticorps fixés anti-protéine antigénique mesurée à l'étape e2),
ledit rapport consistant en la proportion de molécules protéiques porteuses et de molécules de protéine antigénique d'intérêt qui sont liées entre elles par des liaisons covalentes, au sein du produit immunogène de départ.

A l'étape c) du procédé ci-dessus, l'utilisation d'une solution aqueuse de lavage contenant un ou plusieurs agents de dénaturation des protéines entraîne une dénaturation du produit immunogène fixé aux anticorps anti-protéine porteuse, ce qui a pour effet de libérer dans la solution de lavage les molécules de protéine antigénique d'intérêt qui ne sont pas liées par une liaison covalente aux molécules de protéine porteuse. Ainsi, à l'étape d2) du procédé, seules les molécules de protéine antigénique d'intérêt qui sont liées par des liaisons covalentes aux molécules de protéine porteuse sont quantifiées.

De préférence, la solution tampon dénaturante utilisée à l'étape c) contient un agent tensio-actif, tel que le Tween®20, à la concentration finale de 0,1 % v/v.

Aux étapes d1) et d2) les quantités d'anticorps fixés sont de préférence mesurées par incubation des complexes antigènes-anticorps formés à la fin de chacune de ces étapes avec un nouvel anticorps qui est marqué par une molécule détectable, respectivement :
(i) à l'étape d1), un nouvel anticorps dirigé contre l'anticorps anti-protéine porteuse et marqué par une molécule détectable ;
(ii) à l'étape d2), un nouvel anticorps dirigé contre l'anticorps anti-protéine antigénique d'intérêt et marqué par une molécule détectable .

La molécule détectable est indifféremment une molécule radioactive, une molécule fluorescente ou une enzyme. Comme enzyme, on peut utiliser notamment la peroxydase, dont la présence est révélée par colorimètre après incubation avec le substrat ortho-phénylènediamine (OPD).

Un protocole détaillé du procédé ci-dessus est décrit dans les exemples.

A titre illustratif, on a montré selon l'invention, à l'aide du premier ou du second procédé de quantification décrits ci-dessus que :
- dans le produit immunogène comprenant les hétérocomplexes entre la molécule porteuse KLH et des molécules d'interféron alpha humain, de 3% à 8% des molécules d'interféron alpha sont liées de manière covalente à la molécule protéique porteuse KLH ;
- dans le produit immunogène comprenant les hétérocomplexes entre la molécule protéique porteuse KLH et des molécules d'IL-4 murine, environ 11 % des molécules d'IL-4 sont liées de manière covalente à la molécule protéique porteuse KLH.
Il va sans dire que, selon les préparations, le pourcentage de molécules de protéine antigénique d'intérêt liées par une liaison covalente aux molécules de protéines porteuses peut varier significativement. Toutefois, dans tous les cas, ce pourcentage est toujours inférieur à 40%.

L'invention a pour objet un produit immunogène stable pour l'induction d'anticorps à l'encontre d'une ou plusieurs protéines antigéniques chez un sujet, caractérisé en ce qu'il consiste en des hétérocomplexes immunogènes protéiques constitués d'associations entre (i) des molécules de protéines antigéniques et (ii) des molécules protéiques porteuses et en ce que au moins 1 pour cent et en ce que moins de 40 pour cent des protéines antigéniques (i) sont liées par une liaison covalente avec les molécules protéiques porteuses (ii), et en ce que la ou les protéines antigéniques (i) consistent en des cytokines produites naturellement par ledit sujet.

L'invention décrit aussi un produit immunogene stable comprenant des hétérocomplexes immunogènes protéiques pour l'induction d'anticorps à l'encontre d'une ou plusieurs protéines antigéniques chez un sujet, chaque hétérocomplexe comprenant (i) une pluralité de protéines antigéniques, liées à (ii) une molécule protéique porteuse, caractérisé en ce que, dans ledit produit immunogène, au moins 1 pour cent et en ce que moins de 40 pour cent des protéines antigéniques (i) sont liées par une liaison covalente avec les molécules protéiques porteuses (il).

De manière tout à fait préférée, les anticorps dont la production est induite par le produit immunogène de l'invention consistent en des anticorps « neutralisants » ou « bloquants ». Un anticorps « neutralisant » ou un anticorps « bloquant » est défini, selon l'invention, comme un anticorps dont la fixation sur la protéine native cible bloque l'activité biologique de cette protéine native, ce qui est un objectif important recherché par l'invention, lorsque la protéine native à l'encontre de laquelle les anticorps sont dirigés possède une activité biologique délétère pour l'organisme, dans le contexte pathologique visé d'un individu à traiter, par exemple lorsque la protéine native possède une activité angiogénique, une activité immunosuppressive ou encore une activité allergénique, notamment une activité d'induction de la production d'interleukine-4.

Par « molécule protéique porteuse », incluse dans le produit immunogène de l'invention, on entend toute protéine ou peptide d'au moins 15 acides aminés de longueur, quelle que soit sa séquence en acides aminés, et qui, en s'associant de manière partiellement covalente aux molécules d'antigène d'intérêt pour former les hétérocomplexes protéiques constitutifs du produit immunogène de l'invention, permet la présentation d'un grand nombre de molécules d'antigène d'intérêt aux lymphocytes B.

Selon un premier aspect, une molécule protéique porteuse consiste en une protéine ou un peptide d'au moins 15 acides aminés de longueur, ou encore en un oligomère d'un tel peptide, comprenant un ou plusieurs épitopes T auxiliaires ("helper") capables d'activer les lymphocytes T auxiliaires (T helper") de l'organisme hôte pour la production de cytokines, y compris l'interleukine 2, cytokines qui vont à leur tour activer et induire la prolifération des lymphocytes B lesquels, après maturation, vont produire des anticorps à l'encontre de la protéine antigénique (i).

Selon un second aspect, une molécule protéique porteuse consiste en une protéine ou un peptide d'au moins 15 acides aminés de longueur, ou encore en un oligomère d'un tel peptide, comprenant outre un ou plusieurs épitopes T auxiliaires ("helper") décrit dans le premier aspect ci-dessus, un ou plusieurs épitopes T-cytotoxiques capables d'induire une réponse immunitaire cellulaire par production de lymphocytes T-cytotoxiques spécifiques de la molécule protéine porteuse, ces lymphocytes étant capables de reconnaître spécifiquement des cellules exprimant à leur à leur surface ladite protéine porteuse ou tout peptide qui en est dérivé, en association avec des molécules classes du Complexe Majeur d'Histocompatibilité (MHC). Le cas échéant, la molécule protéique porteuse consiste en un oligomère d'une protéine ou d'un peptide comprenant outre un ou plusieurs épitopes T helper, un ou plusieurs épitopes T-cytotoxiques définies ci-dessus.

Selon un troisième aspect, une molécule protéique porteuse consiste en une protéine ou un peptide d'au moins 15 acides aminés de longueur, ou encore en un oligomère d'un tel peptide, comprenant outre un ou plusieurs épitopes T auxiliaires ("helper") définit dans le premier aspect, un ou plusieurs épitopes B, capables d'induire la production d'anticorps par des lymphocytes dirigés contre la protéine porteuse.

Dans certains modes de réalisation, la protéine porteuse, outre sa fonction T helper, utilisée pour activer un réponse anticorps contre l'antigène d'intérêt, peut aussi activer une réponse cytotoxique contre les cellules porteuses de peptides du carrier et/ou stimuler une réponse anticorps contre la molécule protéique porteuse.

La molécule protéique porteuse peut aussi consister en un homo-oligomère ou homo-polymère d'une protéine native dont elle dérive ou encore en un homo-oligomère ou homo-polymère d'un fragment peptidique de la protéine native dont elle dérive. La molécule protéique porteuse peut aussi peut aussi consister en un hétéro-oligomère ou en un hétéro-polymère comprenant une combinaison de plusieurs fragments peptidiques distincts initialement inclus dans la protéine native dont elle dérive.

Par « protéine antigénique», on décrit toute protéine ou tout peptide d'au moins 10 acides aminés de longueur, y compris un peptide haptène, susceptible d'être reconnue spécifiquement par les récepteurs pour l'antigène exprimés par les lymphocytes B d'un organisme hôte, humain ou animal, particulièrement tout mammifère, laquelle protéine antigénique, une fois incluse dans un produit immunogène de l'invention, stimule la production d'anticorps reconnaissant ladite protéine antigénique.

Par « protéine antigénique », on décrit aussi toute protéine comprenant un ou plusieurs épitopes B de la d'une protéine antigénique, native à l'encontre de laquelle la production d'anticorps est recherchée. Ladite molécule d'intérêt antigénique peut consister en la protéine native elle-même ou un dérivé protéique de la protéine native, tels qu'un fragment peptidique de la protéine native, ou encore toute forme biologiquement inactivée de la protéine native obtenue par traitement chimique, physique ou par mutation génétique. La protéine d'intérêt antigénique peut aussi consister en un homo-oligomère ou homo-polymère de la protéine native ou encore en un homo-oligomère ou homo-polymère d'un fragment peptidique de la protéine native. La protéine d'intérêt antigénique peut aussi peut aussi consister en un hétéro-oligomère ou en un hétéro-polymère comprenant une combinaison de plusieurs fragments peptidiques distincts initialement inclus dans la protéine native.

Dans un produit immunogène selon l'invention, avantageusement moins de 30 pour cent, et de préférence moins de 20 pour cent des protéines antigéniques (i) sont liées par une liaison covalente avec les molécules protéiques porteuses (ii).

Dans un produit immunogène selon l'invention, au moins 1 pour cent, et de préférence au moins 2 pour cent des protéines antigéniques (i) sont liées avec les molécules porteuses (ii) par une liaison covalente.

On a montré qu'un produit immunogène de l'invention, tel que défini ci-dessus, était stable en solution aqueuse. La stabilité d'un produit immunogène de l'invention est notamment caractérisée en ce que ledit produit immunogène possède un point isoélectrique propre, distinct du point isoélectrique d'au moins l'un de ses constituants protéiques, respectivement la protéine antigénique (i) et la molécule protéique porteuse (ii), et qu'il migre en conséquence selon une bande protéique distincte d'au moins l'une des bandes protéiques correspondant respectivement aux deux éléments protéiques qui le constituent dans des essais d'isoélectrofocalisation.

On a aussi observé , par des essais d'immuno-empreintes (« Western blot »), que le produit immunogène de l'invention migrait en gel d'électrophorèse, en conditions non dénaturantes, selon une seule bande protéique, ce qui illustre le fait que ledit produit immunogène se présente sous la forme d'une population homogène de constructions protéiques solubles.

De plus, on a montré que la protéine antigénique (i) ainsi que la molécule protéique (ii), incluses sous forme d'hétérocomplexes protéiques dans le produit immunogène de l'invention, étaient toutes les deux reconnues par des anticorps reconnaissant spécifiquement chacune de ces protéines. Ainsi, le produit immunogène selon l'invention comprend la protéine antigénique (i) et la molécule protéique porteuse (ii) dans leur conformation native. Cette caractéristique technique du produit immunogène selon l'invention est particulièrement avantageuse pour l'induction d'une réponse immunitaire à l'encontre des antigènes natifs, c'est-à-dire d'une réponse immunitaire efficace et réellement protectrice de l'organisme hôte. On a montré en particulier qu'un produit immunogène selon l'invention induisait, dans l'organisme hôte dans lequel il était administré, l'induction d'une forte réponse humorale efficace à l'encontre des antigènes natifs accompagnée de la production d'anticorps neutralisants ou bloquants vis-à-vis de l'activité biologique délétère de ces antigènes natifs.

On a montré selon l'invention, avec divers antigènes d'intérêt, que la réponse immunitaire humorale obtenue avec un produit immunogène tel que défini ci-dessus était supérieure de 10 à 1000 fois à la réponse immunitaire humorale obtenue avec une administration d'un conjugué covalent classique entre l'antigène d'intérêt et la molécule protéique porteuse.

De préférence, dans un hétérocomplexe immunogène inclus dans le produit immunogène de l'invention, la pluralité de protéines antigéniques (i) est constituée d'une pluralité d'exemplaires d'une protéine antigénique unique.

Ainsi, selon un mode de réalisation tout à fait préféré, le produit immunogène de l'invention est mis en oeuvre pour l'obtention d'anticorps spécifiques à l'encontre d'un seul antigène d'intérêt.

On a aussi montré selon l'invention qu'un produit immunogène comprenant des hétérocomplexes immunogènes tels que définis ci-dessus était particulièrement bien adapté à l'immunisation d'un individu, par la production d'anticorps, à l'encontre d'un antigène d'intérêt du « soi » (« self antigen »), c'est à dire à l'encontre d'une protéine produite normalement par ledit individu, pour laquelle il existe une tolérance du système immunitaire, en particulier une délétion au moins partielle des clones de lymphocytes T auxiliaires (cellules T helper) reconnaissant spécifiquement ledit antigène.

En d'autres termes, la présentation d'un antigène du « soi » aux cellules du système immunitaire dudit individu, sous la forme du produit immunogène comprenant des hétérocomplexes immunogènes de l'invention, permet de « casser » la tolérance du système immunitaire de l'individu vis-à-vis de cet antigène. Sans vouloir être lié par une quelconque théorie, le demandeur pense que la possibilité d'obtenir un haut niveau de réponse anticorps à l'encontre d'un antigène du « soi » est due à la présence, au sein de l'hétérocomplexe, de nombreux épitopes de type « T auxiliaire » (ou T helpér) portés par la molécule protéique porteuse, qui activent les lymphocytes T auxiliaires, et que les diverses cytokines produites par les lymphocytes T auxiliaires activés, notamment l'IL-2, permet de promouvoir une activation des cellules B à des antigènes du « soi » présentes à l'état dormant dans l'organisme, et de rompre ainsi la tolérance immunitaire des cellules B aux antigènes du « soi ».

Ainsi, selon un mode de réalisation préférentiel, le produit immunogène de l'invention est caractérisé en ce que les protéines antigéniques (i) sont constituées d'une pluralité d'exemplaires d'une protéine normalement reconnue comme une protéine du soi par les cellules du système immunitaire dudit sujet.

Du fait que la plus grande proportion, plus de 60 pour cent, des associations entre l'antigène d'intérêt et la molécule protéique porteuse sont réalisées par des interactions non covalentes, il n'existe aucune limitation théorique dans le nombre de molécules d'antigène d'intérêt associées à une unique molécule protéique porteuse, autre que l'accessibilité stérique des molécules d'antigène d'intérêt à cette molécule porteuse. En particulier, le nombre de molécules d'antigène d'intérêt associées à une même molécule protéique porteuse n'est pas limitée par le nombre de fonctions chimiquement réactives portées par la molécule porteuse permettant la création de liaisons covalentes avec une pluralité de molécules de l'antigène d'intérêt. En conséquence, la seule limite physique semble être le nombre de sites de la molécule protéique porteuse (ii) qui sont accessibles à la protéine antigénique (i).

Pour les mêmes raisons, la taille de l'antigène d'intérêt à associer à la molécule protéique porteuse n'est pas non plus strictement limitée, l'antigène d'intérêt pouvant en conséquence consister en des protéines entières d'au moins 10 kDa, telles les différentes cytokines comme l'IL-4, l'IL-10, le VEGF ou encore l'interféron alpha.

En outre, même pour des antigènes d'intérêt consistant en des protéines entières d'une masse moléculaire supérieur à 10 kDa, un hétérocomplexe immunogène de l'invention peut comprendre une association de plusieurs antigènes d'intérêt sur une seule molécule porteuse, si la taille de la protéine porteuse le permet.

Lorsque la molécule protéique porteuse est de petite taille, par exemple d'une taille inférieure à 10 kDa, ou encore inférieure à 5 kDa, le demandeur pense, sans vouloir être lié par une quelconque théorie, que les associations partiellement covalentes entre l'antigène d'intérêt et ladite protéine porteuse, qui forment les hétérocomplexes protéiques inclus dans le produit immunogène de l'invention, permettent une conformation des hétérocomplexes telle qu'à la fois l'antigène d'intérêt et la molécule protéique porteuse sont accessibles aux récepteurs des cellules du système immunitaire.

Il s'agit même d'un avantage technique supplémentaire procuré par le produit immunogène comprenant des hétérocomplexes tels que définis ci-dessus, puisque la présentation aux lymphocytes B d'une pluralité d'exemplaires d'un antigène d'intérêt sur une même molécule porteuse, comprise dans l'hétérocomplexe, favorise le phénomène de « capping » par « cross-linking » des récepteurs de la cellule B reconnaissant l'antigène, ce qui contribue à l'activation de la cellule B qui reçoit , par ailleurs, les signaux d'activation provenant des cytokines produites par les lymphocytes T auxiliaires activés grâce aux épitopes T auxiliaires portés par la molécule protéique porteuse.

Ainsi, selon un mode de réalisation tout à fait préféré du produit immunogène, celui-ci comprend de 5 à 50 protéines antigéniques (i) pour une molécule protéique porteuse (ii), de préférence de 20 à 40 protéines antigéniques (i) pour une molécule protéique porteuse (ii).

Le nombre de molécules d'antigène d'intérêt sur une seule molécule protéique porteuse dépend respectivement de la taille de la molécule porteuse et de la taille de la molécule d'antigène d'intérêt. Plus la molécule porteuse est grosse et offre une grande surface d'association avec l'antigène d'intérêt, et plus l'hétérocomplexe immunogène comprendra, pour une seule des molécules porteuses qu'il contient, un nombre élevé d'exemplaires de la molécule d'antigène d'intérêt. De même, plus la taille de la molécule d'antigène d'intérêt est réduite, plus le nombre d'exemplaires de la molécule d'antigène d'intérêt sur la même molécule porteuse est grand.

A titre illustratif, on a montré selon l'invention que lorsque la molécule protéique porteuse est le KLH, de 20 à 40 molécules d'IL-4, d'IL-10, d'interféron alpha ou de VEGF sont associés avec chaque molécule porteuse.

On a montré que la solubilité du produit immunogène dans une solution aqueuse varie avec la modification des équilibres régissant les interactions moléculaires au sein des hétérocomplexes, en particulier les équilibres électrochimiques qui dépendent de liaisons dites « faibles » (non covalentes) ou encore les concentrations respectives des protéines antigéniques et de la molécule protéique porteuse, ainsi qu'avec les conditions de force ionique, de pH et de température.

De préférence, les liaisons covalentes entre une ou plusieurs des protéines antigéniques (i) et la molécule protéiques porteuse (ii) sont réalisées par l'intermédiaire d'un agent chimique de liaison bifonctionnel.

Un tel agent chimique peut être le bromure de cyanogène, le glutaraldéhyde, le carbodiimide ou l'anhydride succinique.

En tant que carbodiimides, on peut utiliser par exemple les composés suivants : 1-cyclohexyl-3-(2-morpholinyl-(4-ethyl)carbodiimide (CMC), 1-ethyl-3-(3-dimethyaminopropyl)carbodiimide (EDC) and 1-ethyl-3-(4-azonia-4,4-dimethylpentyl)carbodiimide, 1-cyclohexyl-3-(2-morpholinyl-(4-ethyl)carbodiimide,(1-ethyl-3-(3-dimethya minopropyl carbodiimide (EDC) et 1-ethyl-3-(4-azonia-4,4-dimethylpentyl)carbodiimide.

En tant qu'agents de couplage homo-bifonctionnels, on peut citer les composés suivants :
- esters de N-hydroxysuccinimide, de dithiobis (succinimidylpropionate), disuccinimidyl suberate, et disuccinimidyl tartrate; les imidoesters bifonctionnels dimethyl adipimidate, diméthyl pimelimidate, et diméthyl suberimidate;
- les agents réactifs avec un sulphydryl, 1,4-di-[3'-(2'-pyridyldithio)propionamido]butane, bismaleimidohexane, et bis-N-maleimido-1, 8-octane;
- les halogénures bifonctionnels de type aryle 1,5-difluoro-2,4-dinitrobenzene et 4.4'-difluoro-3,3'-dinitrophenylsulfone;
- le SMCC (succinimidyl-4-(N-maleimidomethyl)cyclohexane-1-carboxylate) ;
- le SIAB (N-succinimidyl(4-iodoacetyl)aminobenzoate),
- le SMPB (succinimidyl-4-(p-maleimidophenyl)butyrate),
- le GMBS (N-(.gamma.-maleimidobutyryloxy)succinimide ester),
- le MPBH (4-(4-N-maleimidopohenyl) acide butyrique hydrazide) ;
- le M2C2H (4-(N-maleimidomethyl)cyclohexane-1-carboxyl-hydrazide),
- le SMPT (succin-imidyloxycarbonyl-.alpha.-methyl-.alpha.-(2-pyridyldithio)toluène) ; et le
- SPDP (N-succinimidyl 3-(2-pyridyldithio)propionate).

Préférentiellement, l'agent chimique de liaison utilisé comprend au moins deux fonctions aldéhyde réactives.

De manière tout à fait préférée, l'agent chimique de liaison est le glutaraldéhyde.

Après formation du produit comprenant les hétérocomplexes protéiques grâce au couplage des molécules protéiques porteuses avec les protéines antigéniques à l'aide de l'agent chimique de liaison, le produit obtenu est stabilisé grâce à un agent de stabilisation des protéines, le formaldéhyde, susceptible de créer des liaisons intrachaine.

Les produits immunogènes comprenant des hétérocomplexes immunogènes de l'invention se présentent sous la forme de microparticules solubles en solution, en particulier en solution aqueuse, dont la taille moyenne varie selon (i) la taille de la molécule protéique porteuse, (ii) la taille et le nombre de protéines antigéniques associées à une même molécule protéique porteuse et (iii) le nombre molécules porteuses associées aux protéines antigéniques présentes dans une particule d'hétérocomplexe.

On a observé que les microparticules d'hétérocomplexes décrites dans les exemples avaient une taille moyenne variant de 100 nm à 300 nm.

Un produit immunogène comprenant des hétérocomplexes protéiques immunogènes de l'invention comprend exclusivement des molécules porteuses associées aux protéines antigéniques, à l'exclusion de tout autre matériau. En particulier, un hétérocomplexe de l'invention ne comprend aucun matériau polymère, protéique ou non protéique, autre que les protéines porteuses et antigéniques qui le caractérisent.

Récemment, ZAGURY D et al. (2001, Proc. Natl. Acad. Sci. USA, 98(14) : 8024-8029), dans une étude bibliographique, suggèrent d'induire une immunité anti-cytokine chez les patients afin de contrecarrer la production anormale dans ces pathologies de certaines cytokines, notamment des interleukines, lymphokines, monokines, interférons qui agissent physiologiquement dans les tissus, localement comme facteur de prolifération, de différenciation ou de mort programmée cellulaire.

Ces auteurs précisent que les stratégies de thérapeutique vaccinale ont été, jusqu'à aujourd'hui, exclusivement ciblées sur l'agresseur antigénique, que ce soit un microorganisme, une cellule ou un allergène, mais n'ont jamais recherché à combattre la dérégulation des cytokines induites sous l'effet de l'agresseur. Ces auteurs proposent une vaccination anti-cytokine en tant que préalable à une vaccination conventionnelle dont le but serait de neutraliser ou de bloquer les effets immunotoxiques du stroma, et de permettre le déroulement normal de la réaction immunitaire adaptée contre l'agresseur antigénique.

De plus, des travaux antérieurs de la demanderesse, relatés dans la demande Internationale publiée sous le n°WO 00/03732, ont montré que, dans le cas de la leucémie ATL, du cancer du col utérin, et du sarcome de Kaposi, respectivement trois protéines étaient impliquées dans une immunosuppression locale au niveau de tumeurs ou de cellules infectées par le VIH1:
la protéine Tax du virus HTLV 1,
la protéine E7 du papillomavirus et
la protéine Tat du virus VIH-1.

La demanderesse avait aussi décrit que certaines de ces protéines immunosuppressives, telles la protéine Tat du HIV1 et la protéine E7 de HPV (souches 16 et 18) ont également des effets activateurs sur les cellules endothéliales vasculaires.

Elle avait en conséquence proposé la mise au point de vaccins anti-cancer ou anti-viraux comprenant un composé immunogène dérivé détoxiqué d'une protéine provenant de cellules cancéreuses, de cellules infectées par un virus ou de cellules immunitaires stromales, initialement immunosuppressive et/ou angiogénique à action locale, comme par exemple une protéine dérivée de la protéine Tat du virus HIV1, la protéine Tax d'un virus HTLV1, la protéine E7 d'un papillomavirus ou encore une lectine mannane-dépendante, sous une forme inactivée.

Or, il est montré selon l'invention que le produit immunogène comprenant des hétérocomplexes immunogènes tels que définis ci-dessus permet l'induction d'une forte réponse anticorps à l'encontre des différentes molécules antigéniques délétères ci-dessus.

Selon un premier aspect, dans l'hétérocomplexe immunogène de l'invention, la ou les protéines antigéniques (i) consistent en des cytokines produites naturellement par ledit sujet.

De manière préférée, la ou les protéines antigéniques (i) sont choisies parmi l'interleukine-4, l'interféron alpha, l'interféron gamma, le VEGF, l'interleukine-10, le TNF alpha, le TGF béta, l'interleukine-5 et l'interleukine 6.

Selon un aspect décrit, la ou les protéines antigéniques (i) constitutives d'un hétérocomplexe immunogène de l'invention sont des protéines immunosuppressives ou angiogéniques, ou des protéines dérivées de protéines immunosuppressives ou angiogéniques.

De manière préférée, la ou les protéines antigéniques (i) immunosupressives ou angiogéniques sont choisies parmi la protéine E7 d'un papillomavirus, la protéine Tat du virus VIH 1, la protéine Tax d'u virus HTLV 1 ou HTLV 2 et la protéine p53 du soi.

Selon un autre aspect décrit, la ou les protéines antigéniques (i) constitutives d'un hétérocomplexe immunogène de l'invention sont des protéines toxiques à faible dose, pour l'homme ou pour un mammifère non humain. Il s'agit tout particulièrement des diverses protéines qui sont léthales pour l'homme à une dose Inférieure à 1 mg, inférieure à 100 µg, inférieure à 10µg, voire inférieure à 1 µg. Il s'agit en priorité des protéines toxiques susceptibles d'être utilisées pour la fabrication d'armes dites « biologiques », telles que la ricine, les toxines botuliques, les entérotoxines de staphylocoque, ou encore une protéine toxique d'anthrax (EF, LF, PA).

La molécule protéique porteuse (ii) incluse dans un hétérocomplexe protéique immunogène de l'invention peut être une molécule porteuse utilisée conventionnellement en immunologie, telle que le KLH, l'ovalbumine, l'albumine sérique bovine (ASB), le Tétanos toxoïde, la toxine cholérique B, etc.

De plus, dans un produit immunogène de l'invention, la molécule porteuse protéique peut être choisie de manière à induire ou à stimuler, outre la production de lymphocytes T helper, une réponse immunitaire cytotoxique et/ou humorale à l'encontre d'elle-même et de sa contrepartie de protéine native dans l'organisme hôte, respectivement par l'activation de lymphocytes T cytotoxiques et de lymphocytes B spécifiques de cette molécule porteuse.

Ce mode de réalisation particulier d'un produit immunogène de l'invention est particulièrement utile lorsque l'on recherche simultanément une réponse anticorps efficace à l'encontre d'une protéine délétère immunosuppressive ou angiogénique, notamment par la production d'anticorps neutralisants ou bloquants, et une réponse immunitaire cellulaire effectuée par des lymphocytes T cytotoxiques dirigés contre des cellules présentant à leur surface l'antigène natif associé aux molécules de classe I du Complexe Majeur d'Histocompatibilité (CMH), par exemple un antigène d'un pathogène comme le virus VIH1 ou un Papillomavirus, ou un antigène spécifiquement exprimé dans les cellules cancéreuses, comme le CEA, la p53, le Di12, etc.

Ainsi, selon ce mode de réalisation particulier, le produit immunogène de l'invention est caractérisé en ce que la molécule protéique porteuse (ii) est une protéine immunogène induisant, outre la production de lymphocytes T helper, la production de lymphocytes T cytotoxiques dirigés à l'encontre de cellules présentant à leur surface ladite molécule protéique porteuse, ou tout peptide qui en est dérivé, en association avec des molécules de classe I du Complexe Majeur d'Histocompatibilité (MHC), et/ou la production d'anticorps par des lymphocytes B dirigés contre la protéine porteuse.

Ainsi, les produits immunogènes comprenant des hétérocomplexes immunogènes de l'invention constituent des moyens immunologiques efficaces pour la vaccination thérapeutique active d'un individu, qu'il soit un mammifère humain ou un mammifère non humain, à l'encontre d'une grande diversité de pathologies.

Des exemples illustratifs de composition d'hétérocomplexes immunogènes contenus dans un produit immunogène tel que décrit pour prévenir ou traiter, par une vaccination thérapeutique active, des pathologies diverses sont indiqués ci-dessous.

### a) Pour la prévention ou le traitement du SIDA :

- Molécule protéique porteuse (ii) :les protéines gp 120, gp160, p24, p17, nef, ou Tat du virus HIV1, détoxiquées ou stabilisées si cela est nécessaire, des fragments immunogènes de ces protéines ou encore une protéine immunogène qui en est dérivée (Zagury et al., 1998).

On peut aussi utiliser la protéine gp 120 mimotope qui est décrite par Fouts et al. (2000) et par Fouts et al. (2002).
- Protéine antigénique (i): les protéines Tat, IFNα, IL10 et TGFβ, détoxiquée si cela est nécessaire, des fragments immunogènes de ces protéines ou une protéine immunogène qui en est dérivée.

### b) Pour la prévention ou le traitement du cancer du col utérin :

- Molécule protéique porteuse (ii) : les protéines L1, L2 et E7 du papillomavirus, préférentiellement d'un papillomavirus de la souche 16 ou 18, détoxiquée ou stabilisée si cela est nécessaire, des fragments immunogènes de ces protéines ou encore une protéine immunogène qui en est dérivée (Le Buanec et al., 1999).
- Protéine antigénique (i) : les protéines E7, IFNα, IL10, TGFβ, TNFα et VEGF, détoxiquées ou stabilisées si cela est nécessaire, des fragments immunogènes de ces protéines ou une protéine immunogène qui en est dérivée.

### c) Pour la prévention ou le traitement de la leucémie A TL induite par les virus HTLV1 ou 2:

- Molécule protéique porteuse (ii) les protéines gp61 et Tax des virus HTLV1 ou 2, détoxiquée si cela est nécessaire, des fragments immunogènes de ces protéines ou encore une protéine immunogène qui en est dérivée (Cowan et al., 1997 ; Mori et al., 1996).
- Protéine antigénique (i) : les protéines Tax, IL10, IFNα ou TGFβ, TNFα, VEGF détoxiquées, des fragments immunogènes de ces protéines ou encore une protéine qui en est dérivée.

### d) Pour la prévention ou le traitement du cancer du colon :

- Molécule protéique porteuse (ii) : les protéines CEA et p53, détoxiquées si cela est nécessaire, des fragments immunogènes de ces protéines ou encore un protéine immunogène qui en est dérivée (Zusman et al., (1996).
- Protéine antigénique (i) : les protéines IFNα, TGFβ, IL10, FasL et VEGF, détoxiquées, des fragments peptidiques immunogènes de ces protéines ou encore une protéine immunogène qui en est dérivée.

### e) Pour la prévention ou le traitement du cancer du sein :

- Molécule protéique porteuse (ii) :la protéine Di12, des fragments immunogènes de cette protéine ou encore une protéine qui en est dérivée (Yoshiji et al., 1996).
- Protéine antigénique (i) : les protéines TGFβ, TNFα et VEGF, détoxiquées si nécessaire, des fragments immunogènes de ces protéines ou encore une protéine immunogène qui en est dérivée.

### f) Pour la prévention ou le traitement du cancer du pancréas :

- Molécule protéique porteuse (ii) :la protéine CaSm, détoxiquée si cela est nécessaire, des fragments immunogènes de cette protéine ou encore un protéine immunogène qui en est dérivée.
- Protéine antigénique (i) : les protéines VEGF et TNFα, détoxiquées ou stabilisées si nécessaire, des fragments immunogènes de ces protéines ou encore une protéine immunogène qui en est dérivée.

### g) Pour la prévention ou le traitement du cancer de la prostate :

- Molécule protéique porteuse (ii) : les protéines OSA et ETS2, détoxiquées ou stabilisées si nécessaire, des fragments immunogènes de ces protéines ou encore une protéine immunogène qui en est dérivée. (Sementchenko VI et al., 1998).
- Protéine antigénique (i) : les protéines IL6 et TGFβ, détoxiquées ou stabilisées si nécessaire, des fragments immunogènes de ces protéines ou encore une protéine immunogène qui en est dérivée. (Adler et al., 1999).

### h) Pour la prévention ou le traitement de certaines allergies :

- Molécule protéique porteuse (ii) est choisi parmi les allogènes moléculaires, tels que les protéines Bet v 1 (pollen de bouleau), Der p 1 (acarien) et Fel d 1 (chat), leurs fragments peptidiques immunogènes ou encore une protéine immunogène qui en est dérivée. L'antigène Bet v 1 est décrit notamment par Ferreira et al. (1993), l'antigène Der p 1 est décrit notamment par Tovey et al. (1981) et l'antigène Fel d 1 est décrit notamment par Morgenstern et al. (1991)
- Protéine antigénique (i) : elle induit la production d'anticorps neutralisants ou bloquants à l'encontre du facteur cytokinique IL4, qui est produit principalement par les lymphocytes T de type Th2, qui orientent la réponse immunitaire humorale vers la production d'anticorps d'isotype IgE. Selon un autre mode de réalisation, la protéine antigénique (i) induit la production d'anticorps neutralisants ou bloquants à l'encontre du facteur cytokinique IL5, qui est produit principalement par les lymphocytes T de type Th2.

Selon encore un autre mode de réalisation, la prévention de l'allergie peut être réalisée à l'aide d'un produit immunogène induisant une réponse anticorps à l'encontre du principal effecteur de la granulation des basophiles, les anticorps d'isotype IgE. Dans ce but, l'invention fournit un produit immunogène comprenant (i) un anticorps humain d'isotype IgE et (ii) la protéine Tat Inactivée du VIH1.

### i) Pour la prévention contre les protéines léthales utilisées dans les armes biologiques.

Egalement, un produit immunogène tel que décrit peut être utilisé pour immuniser des individus à l'encontre de nombreux produits toxiques utilisés notamment dans les armes chimiques et biologiques, comme par exemple la ricine.
Parmi les protéines les plus toxiques à l'encontre desquelles une immunisation, principalement par la production d'anticorps, est recherchée, on préfère les toxines botuliques, la ricine, les entérotoxines de staphylocoque, les toxines de *Clostridium perfringens* et les protéines toxiques de l'anthrax.

De manière générale, pour réaliser un produit immunogène tel que décrit dans lequel la protéine antigénique (i) est une protéine fortement toxique du type ci-dessus, on utilise une protéine préalablement détoxifiée sous la forme d'un toxoïde. Pour détoxifier la protéine, avant son utilisation pour préparer un produit immunogène selon l'invention, on peut utiliser diverses méthodes, et de préférence l'une des méthodes suivantes :
a) traitement de la protéine toxique native par le glutaraldéhyde ;
b) traitement de la protéine toxique native par action combinée du formol et du glutaraldéhyde ; ou
c) le cas échéant, par modification chimique des groupements His et Tyr à l'aide de réactifs appropriés, par exemple par carboxyméthylation de ces résidus d'acides aminés.

Pour la prévention de l'action léthale de toxines provenant de *Bacillus anthracis*, on utilise de préférence, comme protéines antigéniques (i), une protéine détoxifiée provenant d'une toxine d'anthrax choisie parmi les protéines EF (« Edema Factor »), LF (« Lethal Factor ») et PA (« Protective Antigen »).

Pour la prévention de l'action léthale de toxines provenant de *Clostridium perfringens*, on utilise de préférence, comme protéine antigénique (i), une protéine détoxifiée provenant de la toxine Epsilon de *Clostridium perfringens.*

Pour la prévention de l'action léthale de toxines provenant de *Clostridium botulinum,* on utilise de préférence, comme protéines antigéniques (i), une protéine détoxifiée provenant d'une toxine botulique choisie parmi les toxines A, B, C, D, E, F et G, qui sont naturellement synthétisées sous la forme d'une chaîne polypeptidique unique de 150 kDa, ou encore le fragment H_{c} des toxines botuiliniques, lequel fragment H_{c} possède une masse moléculaire d'environ 50 kDa.

Pour produire les toxines botuliques inactivées, l'homme du métier peut utiliser toute techniques connue en soi, notamment celles utilisées pour préparer des compositions vaccinales antérieures, telles que celles décrites par Fiock et al. ou par Siegel et al. (Fiock, M.A., Cardella, M.A., Gearinger, N.F., J. Immunol., 1963, 90, 697-702; Siegel, L.S., J. Clin. Microbiol., 1988, 26, 2351-2356).

Pour la prévention de l'action léthale de toxines provenant de la graine de ricin *(Ricinus communis),* on utilise de préférence, comme protéine antigénique (i), une protéine détoxifiée provenant de la toxine du ricin, de préférence le fragment β de la ricine.

Ainsi, l'invention décrit aussi un produit immunogène comprenant (i) le fragment β de la ricine et (ii) la protéine KLH.

Pour purifier la ricine, l'homme du métier peut utiliser toute technique connue, telles que celles décrites par Osborne et al., Kabat et al. ou Kunnitz et al. (Osborne,T.B., Mendel,L.B. and Harris,J.F.: Amer.J.Physiol.,1905,14,259-269; Kabat,E.A.Heidelberger,M. and Bezer,A.E.:J.Biol.Chem.,1947,168,629- ; Kunnitz,M. and McDonald,M. :J.Gen.Physiol.,1948,22,25-Moulé,Y.:Bull.Soc.Chim.Biol.,1951,33,1461-1467). Il peut aussi utiliser les techniques de purification par chromatographie d'affinité décrites par Tomila et al., Nicolson et, al. ou Olsnes et al. (Tomila,M.,Kurokawa,T., Onozaki,K. et al.: Experientia,1972,28;84-85; Nicolson,G.L. and Blaustein,J.:J.Biochim.Biophys.Acta,1972,266,543-547; Olsnes,S. Salvedt,E. and Pihl,A.:J.BioLChem.,1974,249,803-810). Les chaînes A et B de la ricine peuvent être purifiées comme décrit par Hedge et al. *(*Hedge,R. and Podder,S.K.,:Eur.Biochem.,1998,254, 596-601).

Pour la prévention de l'action léthale de toxines provenant de staphylocoque, et plus particulièrement de *Staphylococcus aureus,* on utilise de préférence, comme protéine antigénique (i), une protéine détoxifiée provenant d'une toxine choisie parmi la SEA (« Staphylococcal Enterotoxin A »), la SEB (« Staphylococcal Enterotoxin B »), la SEC (« Staphylococcal Enterotoxin C »), la SED (« Staphylococcal Enterotoxin D »), la SEE (« Staphylococcal Enterotoxin E »), la SEG (« Staphylococcal Enterotoxin G »), la SEH (« Staphylococcal Enterotoxin H »), la SEI (« Staphylococcal Enterotoxin I ») et la TSST-1 (« Toxic Shock Syndrome Toxin-1 »).,

Les entérotoxines ci-dessus peuvent être préparées par l'homme du métier à l'aide des techniques décrites dans les travaux référencés ci-dessous, en relation avec la description de chacune de ces toxines.

La SEA est synthétisée sous forme d'une entérotoxine précurseur de 257 aminoacides (Huang, I.Y., Hughes, J.L., Bergdoll, M.S. and Schantz, E.J. : J. Biol. Chem. 1987, 262, 7006-7013). La toxine mature de masse moléculaire égale à 27100 Da dérive de la toxine précurseur par perte d'une séquence leader hydrophobe N-terminale de 24 résidus d'acides aminés (Betley, M.J. and Mekalanos, J.J. : J. Bacteriol., 1998, 170, 34-41). SEA existe sous 3 isoformes différentes par leur PI.

La protéine précurseur de SEB comprend 267 aminoacides (Mr=31 400 Da) avec un peptide signal N-terminal de 27 acides aminés. Son site de fixation au récepteur de cellulles T (« T-Cell Receptor » ou « TCR ») embrasse la cavité de faible profondeur, alors que la molécule de MHC classe II se fixe sur un site adjacent (Kappler, J.W., Herman, A., Clements, J. and Marrack, P.: J. Exp. Med., 1992, 175, 387-396 ; Papageorgiu, A.C., Trauter, H.S. and Acharya, K.R.: J. Mol. Biol., 1998, 277, 61-79 ; Soos, J.M. and Johnson, H.M. : Biochem. Biophys. Res. Commun., 1994, 201, 596-602).

La SEC présentent 3 sous-types SEC 1, SEC 2 et SEC 3 antigéniquement distincts. La protéine précurseur contient 267 résidus d'acides aminés (Houde, C.J., Hackett, S.P. and Bohach, G.A. : Mol. Gen. Genet., 1990, 220, 329-333) avec un peptide signal de 27 résidus d'aminoacides (Bohach, G.A. and Schlievert, P.M. : Infect. Immun., 1989, 57, 2243-2252).

La SED est faite de 258 résidus d'acides aminés avec un peptide signal de 30 résidus AA. Sa structure tridimensionnelle est semblable à la structure des autres superantigènes bactériens.

La SEE, dont la masse moléculaire est de 26000 Da présente 81 % d'homologie de séquence en AA avec SEA.

La SEG est constituée de 233 résidus d'acides aminés (Munson, S.H., Tremaine, M.T., Betley, M.J. and Welch, R.A.: Infect. Immun., 1998, 66, 3337-3348).

La SEH présente une masse moléculaire est de 27300 Da (Su, Y.C. and Wong, A.C. : Apll. Environ. Microbiol., 1995, 61,1438-1443). Elle ne donne pas de réaction immunologique croisée avec les autres entérotoxines.

La SEI a une séquence comprenant 218 résidus d'aminoacides. C'est la toxine qui présente le plus faible niveau d'homologie avec les autres entérotoxines.

La SEJ faite de 269 résidus d'acides aminés présente une homologie de séquence en AA élevée avec SEA, SEE et SED (64-66%).

De préférence, un produit immunogène tel que décrit comprend, en combinaison, plusieurs protéines antigéniques (i) dérivées chacune d'une protéine toxique ci-dessus, par exemple 2, 3, 4 ou 5 protéines antigéniques (i) dérivées chacune d'une protéine toxique ci-dessus.

Par exemple, un produit immunogène tel que décrit pour la préparation d'une composition vaccinale destinée à prévenir la toxicité des entérotoxines de staphylocoque comprend de préférence 2, 3, 4 ou 5 protéines antigéniques (I) dérivées chacune d'une entérotoxine de staphylocoque.

Selon un mode de réalisation particulier d'un produit immunogène tel que décrit dans lequel la ou les protéines antigéniques (i) sont dérivées de protéines hautement toxiques pour l'homme, le protéine porteuse consiste en la protéine KLH.

Ainsi selon un premier aspect particulier d'un produit immunogène tel que décrit, dans lequel la molécule protéique porteuse induit à la fois la production de lymphocytes T auxiliaires (« T helper »), de lymphocytes T cytotoxiques et de lymphocytes B spécifiques de la molécule protéique porteuse, ladite molécule protéique porteuse (ii) est choisie parmi les protéines L1, L2 et E7 d'un Papillomavirus.

Selon un second aspect particulier d'un produit Immunogène de l'invention, dans lequel la molécule protéique porteuse induit, outre la production de lymphocytes T auxiliaires (« T helper »), la différenciation de lymphocytes T cytotoxiques et de lymphocytes B spécifiques de la molécule protéique porteuse, ladite molécule protéique porteuse (ii) est choisie parmi les protéines gp160, p24, p17, Nef et Tat du virus HIV1.

Selon un troisième aspect particulier d'un hétérocomplexe immunogène de l'invention, dans lequel la molécule protéique porteuse induit à la fois la production de lymphocytes T auxiliaires (« T helper ») lymphocytes T cytotoxiques et de lymphocytes B spécifiques de la molécule protéique porteuse, ladite molécule protéique porteuse (ii) est choisie parmi les protéines CEA, p53, Di12, CaSm, OSA et ETS2.

Selon un quatrième aspect particulier d'un produit immunogène de l'invention, dans lequel la molécule protéique porteuse induit, outre la différenciation de lymphocytes T auxiliaires (« T helper ») la production d'anticorps dirigés contre la molécule protéique porteuse, ladite molécule protéique porteuse (ii) est choisie parmi les protéines Bet v 1, Der p 1 et Fel d 1.

Dans un produit immunogène tel que décrit, les hétérocomplexes protéiques immunogènes préférés sont choisis parmi les hétéro complexes suivants, dans lesquels les protéines antigéniques (i), d'une part et la molécule porteuse protéique (ii), d'autre part, sont respectivement :
a) (i) IL-4 et (ii) KLH ;
b) (i) interféron alpha et (ii) KLH ;
c) (i) VEGF et (ii) KLH ;
d) (i) IL-10 et (ii) KLH ;
e) (i) interféron alpha et (ii) gp160 de VIH1
f) (i) IL-4 et (ii) l'antigène allergène Bet v 1 ; et
g) (i) le VEGF et (ii) la protéine E7 d'un Papillomavirus.
h) (i) la protéine Tat inactivée de VIH1 et (ii) la protéine gp 120 de VIH1.
(i) (i) un anticorps humain d'isotype IgE et (ii) la protéine Tat de VIH1 inactivée ;
(j) (i) le fragment β de la ricine et (ii) KLH.

### Procédé de préparation d'un produit immunogène comprenant des hétérocomplexes protéiques immunogènes de l'invention

L'invention a encore pour objet un procédé de préparation d'un produit immunogène comprenant des hétérocomplexes immunogènes tels que définis ci-dessus, caractérisé en ce qu'il comprend les étapes suivantes :
a) incuber les protéines antigéniques (i) consistant en des cytokines produites naturellement par un sujet, et la molécule porteuse (ii) dans un rapport molaire (i) : (ii) de 5:1 à 50:1, en présence d'un agent chimique de liaison;
b) stabiliser les hétérocomplexes formés à l'étape a) par traitement avec le formaldéhyde;
c) récupérer le produit immunogène comprenant les hétérocomplexes immunogènes préparé à l'étape b).

De préférence, l'agent chimique de liaison est le glutaraldéhyde.

De manière tout à fait préférée, le procédé est en outre caractérisé en ce que l'étape a) est suivie d'une étape de stabilisation du produit comprenant les hétérocomplexes immunogènes par le formaldéhyde, préalablement à l'étape c) de récupération des hétérocomplexes.

De préférence, lorsque le glutaraldéhyde est utilisé comme agent chimique de liaison, il est présent dans le milieu réactionnel de couplage à une concentration finale comprise entre 0,002M et 0,03M, avantageusement entre 0,02 M et 0,03M, de préférence à la concentration finale de 0,026M.

La réaction de couplage avec le glutaraldéhyde est avantageusement réalisée, pendant 20 minutes à 60 minutes de préférence 30 minutes, à température allant de 20 à 25°C.

Après l'étape de couplage, le glutaraldéhyde en excès est éliminé, par exemple par dialyse à l'aide d'une membrane de dialyse ayant un seuil de coupure de 3 kDa. L'étape de dialyse est avantageusement réalisée à 4°C, dans un tampon ajusté à pH 7,6.

Pour stabiliser le produit comprenant les hétérocomplexes protéiques préparer à l'étape a), ledit produit peut être traité en solution par le formaldéhyde, par exemple par du formaldéhyde à la concentration finale de 3 mM. La réaction de stabilisation a avantageusement une durée comprise entre 12 et 48 heures, de préférence entre 20 et 30 heures et est de manière tout à fait préférée d'une durée de 24 heures. La réaction de stabilisation par le formaldéhyde est avantageusement stoppée par addition de glycine, de préférence à la concentration de 0,1 M, pendant 1 heure et à une température comprise entre 20 et 25°C.

### Les compositions comprenant un produit immunogène comprenant des hétérocomplexes protéiques immunogènes de l'invention

La présente invention a aussi pour objet une composition comprenant un produit immunogène tel que défini ci-dessus.

L'invention est également relative à une composition pharmaceutique comprenant un produit immunogène protéique tel que défini ci-dessus.

L'invention a encore pour objet une composition immunogène caractérisée en ce qu'elle comprend, à titre de principe actif, un produit immunogène tel que défini ci-dessus, en association avec un ou plusieurs excipients physiologiquement compatibles.

Elle a également trait à une composition vaccinale caractérisée en ce qu'elle comprend, à titre de principe actif, un produit immunogène tel que défini ci-dessus, en association avec un ou plusieurs excipients physiologiquement compatibles.

Selon les objectifs poursuivis, on utilise des adjuvants systémiques ou des adjuvants mucosaux. Par exemple, on utilise de préférence un adjuvant mucosal pour prévenir les cancers des tissus épithéliaux et on utilise de préférence des adjuvants systémiques pour prévenir ou traiter les infections par des virus, tels que par HIV1 et HTLV1, ou encore pour prévenir ou traiter les allergies.

Parmi les adjuvants systémiques, on utilise de préférence les adjuvants de type IFA (Adjuvant Incomplet de Freund), le phosphate de calcium ou l'hydroxyde d'alumine.

Parmi les adjuvants mucosaux, on utilise de préférence des adjuvants comme la chloratoxine B (CTB) ou un mutant de la toxine LT (LTµ).

Selon un aspect particulier, une composition immunogène selon l'invention comprend aussi un ou plusieurs agents immuno-stimulants, en combinaison avec un adjuvant de l'immunité, comme par exemple l'agent immuno-stimulant CpG bien connu dans l'état de la technique.

On a en effet montré selon l'invention que l'utilisation de l'adjuvant CpG, et tout particulièrement l'adjuvant CpG dans lequel les liaisons intrachaîne entre les nucléotides consistent en des liaisons phosphorothioate permettait la stimulation de la production simultanée d'anticorps d'isotypes IgG et IgA, après administration systémique.

Elle a également trait à un vaccin, mucosal ou systémique, caractérisé en ce qu'il comprend, à titre de principe actif, un produit immunogène tel que défini ci-dessus, en association avec un ou plusieurs excipients, dont des adjuvants d'immunité, physiologiquement compatibles.

Les compositions immunogènes ou les vaccins selon la présente invention trouvent leur emploi par exemple dans le traitement tant curatif que préventif des cancers, notamment des cancers induits par des virus comme par exemple, l'ATL (Acute T cell leukemia) causé par le HTLV1, ou le cancer du col utérin causé par le papillomavirus, ou encore le lymphome de Burkitt ou le sarcome de Kaposi causés par des virus de la famille herpès, respectivement l'Epstein-Barr (EBV) et le HHV8 ainsi que dans le traitement du SIDA, ainsi que pour prévenir ou traiter les réactions allergiques.

Les produits immunogènes selon l'invention peuvent être utilisés comme suit :

On administre à un patient, sous une forme adaptée à l'administration systémique ou mucosale, un produit immunogène comprenant des hétérocomplexes protéiques immunogènes selon la présente invention, par exemple par voie intranasale, en quantité suffisante pour être efficace sur le plan thérapeutique, à un sujet ayant besoin d'un tel traitement. La dose administrée peut aller par exemple de 10 à 1000 µg par voie intranasale, une fois par semaine pendant deux mois, puis, compte tenu du caractère transitoire de la réponse anticorps dirigée contre l'antigène d'intérêt, périodiquement en fonction du taux des anticorps sériques, par exemple tous les 2-6 mois.

On pourra administrer dans une même préparation deux ou plusieurs produits immunogènes différents pour induire des anticorps neutralisant tous les sites fonctionnels délétères au cas ou une seule molécule ne porte pas tous les sites actifs de la toxine ou de la cytokine surproduite que l'on veut neutraliser.

A titre de médicaments, les produits immunogènes de l'invention peuvent être incorporés dans des compositions pharmaceutiques destinées à une administration par voie systémique ou bien à une administration par la voie muqueuse, notamment oro-muqueuse, en particulier la voie intranasale, la voie orale et la voie vaginale. L'administration peut avoir lieu en dose unique ou répétée une ou plusieurs fois après un certain intervalle de temps.

C'est pourquoi la présente demande a également pour objet une composition pharmaceutique, curative ou préventive, caractérisée en ce qu'elle comprend à titre de principe actif, un ou plusieurs produits immunogènes tels que définis ci-dessus. Le produit immunogène peut être conditionné seul ou mélangé à un excipient ou mélange d'excipients pharmaceutiquement acceptables tel qu'un adjuvant. Parmi les excipients destinés à la voie intranasale ou orale, on retient particulièrement les capryl caproyl macrogol glycérides comme le Labrasol® de la Société GATTEFOSSE ou l'hydroxyde d'alumine (Alhydragel, Superfos, Danemark).

Pour l'administration orale selon l'invention, on associera le principe actif à un adjuvant d'immunité mucosale tel qu'un mutant de CT, de LT ou de CTB.

On retient tout particulièrement les formes galéniques décrites par Boyaka et al. « Strategies for mucosal vaccine development » dans Am. J. Trop. Med. Hyg. 60(4), 1999, pages 35-45. On peut citer aussi les microgranules gastro résistantes, notamment bioadhésives telles que ceux décrits par Rojas et al dans Pharmaceutical Research, vol.16, n°2, 1999, page 255.

Dans des conditions particulières de mise en oeuvre, on retient une composition pharmaceutique vaccinale ci-dessus, caractérisée en ce qu'elle comprend un adjuvant d'immunité mucosale, tel un mutant de CT (cholera toxine) ou de LT (entérotoxine labile d'*E*. *coli*).

Dans d'autres conditions particulières de mise en oeuvre, on retient une composition pharmaceutique vaccinale ci-dessus, caractérisée en ce qu'elle renferme un adjuvant absorbant le principe actif, tels l'hydroxyde d'alumine où les particules d'or.

La présente invention a encore pour objet un procédé de préparation d'une composition ci-dessus décrite, caractérisé en ce que l'on mélange, selon des méthodes connues en elles-mêmes, le ou les produits immunogènes actifs avec des excipients acceptables, notamment pharmaceutiquement acceptables et le cas échéant, avec un adjuvant d'immunité systémique ou mucosale.

Dans des conditions préférentielles de mise en oeuvre du procédé ci-dessus, on prépare des microgranules bioadhésives et gastrorésistantes pour la voie orale digestive renfermant les principes actifs immunogènes et le cas échéant les adjuvants.

La présente invention est en outre illustrée par les exemples suivants :

### EXEMPLES

### Exemples de préparations d'hétérocomplexes :

### Exemple 1 : préparation de l'hétérocomplexe KLH-VEGF murin

0,58 mg de protéine KLH sont dissous dans 0,5 ml de tampon phosphate 10 mM pH 8,5. A cette solution sont ajoutés 1 mg de VEGF murin dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune effectuées dans un boudin de dialyse dont le seuil de coupure est de 3 kDa, à 4 °C, contre 200 ml de tampon phosphate pH 7,6 10 mM.

Le mélange est alors traité par du formaldéhyde à la concentration finale de 33 mM pendant 24 heures. Puis la réaction est bloquée par addition de glycine 0.1 M finale pendant 1 heure à température ambiante.

Le mélange est enfin dialysé dans les mêmes conditions que la dialyse effectuée précédemment.

### Exemple 2 : Hétérocomplexe KLH-VEGF humain.

Cet hétérocomplexe représente le principe actif d'un vaccin propre à induire principalement chez le vacciné la production d'anticorps qui neutralisent le VEGF humain.

0,58 mg de protéine KLH sont dissous dans 0,5 ml de tampon phosphate 10 mM pH 8,5. A cette solution sont ajoutés 1 mg de VEGF humain dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune, effectuées dans un boudin de dialyse dont le seuil de coupure est de 3 kDa, à 4°C, contre 200 ml de tampon phosphate pH 7,6 10 mM.

Le mélange est alors traité par du formaldéhyde à la concentration finale de 33 mM pendant 24 heures. Puis la réaction est bloquée par addition de glycine 0.1 M finale pendant 1 heure à température ambiante. Le mélange est enfin dialysé dans les mêmes conditions que la dialyse effectuée précédemment.

**Exemple 3 : préparation d'un hétérocomplexe KLH-IL4 murin.**

0,841 mg de protéine KLH sont dissous dans 0,8 ml de tampon phosphate 10 mM pH 8,5. A cette solution sont ajoutés 1 mg de IL4 murin dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune effectuées dans un boudin de dialyse dont le seuil de coupure est de 3 kDa, à 4 °C, contre 200 ml de tampon phosphate pH 7,6 10 mM.

Le mélange est alors traité par du formaldéhyde à la concentration finale de 33 mM pendant 24 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple 4 : préparation d'un hétérocomplexe KLH-IL4 humain.

Cet hétérocomplexe représente le principe actif d'un vaccin propre à induire principalement chez le vacciné la production d'anticorps qui neutralisent l'IL4 humain.

1 mg de protéine KLH sont dissous dans 1 ml de tampon phosphate 10 mM pH 8,5. A cette solution sont ajoutés 1 mg de protéine IL4 murine dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune effectuées dans un boudin de dialyse dont le seuil de coupure est de 3 kDa, à 4 °C, contre 200 ml de tampon phosphate pH 7,6 10 mM.

Le mélange est alors traité par du formaldéhyde à la concentration finale de 33 mM pendant 24 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple 5 : préparation d'un complexe KLH-IFNα.

Ce conjugué représente le principe actif d'un vaccin propre à induire principalement chez le vacciné la production d'anticorps qui neutralisent l'IFNα humain.

0,625 mg de protéine KLH sont dissous dans 0,6 ml de tampon phosphate 10 mM pH 8,5. A cette solution sont ajoutés 1 mg de protéine IFNα humaine dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune effectuées dans un boudin de dialyse dont le seuil de coupure est de 3 kDa, à 4 °C, contre 200 ml de tampon phosphate pH 7,6 10 mM.

Le mélange est alors traité par du formaldéhyde, à la concentration finale de 33 mM pendant 48 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple 6 : préparation d'un complexe gp160-IFNα.

Cet hétérocomplexe représente le principe actif d'un vaccin propre à induire chez le vacciné la production d'anticorps qui neutralisent à la fois la protéine de structure gp160 du virus HIV-1 et la protéine IFNα cytokine immunosuppressive. De plus cet hétérocomplexe doit permettre d'induire une réaction cellulaire (chimiokines, T auxiliaire, CTL) dirigée contre les cellules infectées exprimant la gp160.

0,380 mg de protéine gp160 sont dissous dans 0,380 ml de tampon phosphate 10 mM pH 8,5. A cette solution sont ajoutés 1 mg de protéine IFNα humaine dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune dans un boudin de dialyse dont le seuil de coupure est de 3 kDa, à 4 °C, contre 200 ml de tampon phosphate pH 7,6 10 mM.

Le mélange est alors traité par du formaldéhyde à la concentration finale de 33 mM pendant 48 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple comparatif 7 : preparation d'un hétérocomplexe gp160-Tat toxoïde. (La protéine Tat est inactivée de manière biochimique)

Cet hétérocomplexe représente le principe actif d'un vaccin propre à induire chez le vacciné la production d'anticorps qui neutralisent à la fois la protéine de structure gp160 du virus HIV-1 et la protéine Tat extracellulaire du VIH-1. De plus ce complexe doit permettre d'induire réaction cellulaire (chimiokines, T auxiliaire, CTL) dirigée contre les cellules infectées exprimant la gp160.

0,550 mg de protéine gp120 sont dissous dans 0,550 ml de tampon phosphate 10 mM pH 8,5. A cette solution sont ajoutés 1 mg de protéine Tat toxoïde dissous dans 1 ml du même tampon.

Le mélange de protéine ainsi obtenu est traité par du glutaraldéhyde à la concentration finale de 0,026 M pendant 30 minutes à température ambiante.

Puis la réaction est bloquée par addition de glycine 0.1 M finale pendant 1 heure à température ambiante L'excès de glycine est ensuite éliminé par 3 dialyses successives de 2 heures chacune dans un boudin de dialyse dont le seuil de coupure est de 3 kDa, à 4 °C, contre 200 ml de tampon phosphate pH 7,6 10 mM.

### Exemple comparatif 8 : préparation d'un hétérocomplexe gp160-Tat GM. (La protéine Tat est inactivée de manière génétique)

Cet hétérocomplexe représente le principe actif d'un vaccin propre à induire chez le vacciné la production d'anticorps qui neutralisent à la fois la protéine de structure gp160 du virus HIV-1 et la protéine Tat de régulation du VIH-1. De plus cet hétérocomplexe doit permettre d'induire réaction cellulaire (chimiokines, T auxiliaire, CTL) dirigée contre les cellules infectées exprimant la gp160.

0,550 mg de protéine gp160 sont dissous dans 0,550 ml de tampon phosphate 10 mM pH 8,5. A cette solution sont ajoutés 1 mg de protéine Tat GM dissous dans 1 ml du même tampon.

Le mélange de protéine ainsi obtenu est traité par du glutaraldéhyde à la concentration finale de 0,026 M pendant 30 minutes à température ambiante.

Puis la réaction est bloquée par addition de glycine 0.1 M finale pendant 1 heure à température ambiante. L'excès de glycine est ensuite éliminé par 3 dialyses successives de 2 heures chacune dans un boudin de dialyse dont le seuil de coupure est de 3 kDa, à 4 °C, contre 200 ml de tampon phosphate pH 7,6 10 mM.

### Exemple 9 : Préparation d'un hétérocomplexe KLH-TNFa murin

Ce conjugué représente le principe actif d'un vaccin propre à induire principalement chez le vacciné la production d'anticorps qui neutralisent le TNFα murin.

0,625 mg de protéine KLH sont dissous dans 0,6 ml de tampon borate 10 mM pH 8,8 150 mM NaCl. A cette solution sont ajoutés 1 mg de protéine IFNα humaine dissous dans 1 ml du même tampon.

Le mélange de protéine ainsi obtenu est traité par du glutaraldéhyde à la concentration finale de 0,026 M pendant 45 minutes à température ambiante.

L'excès de glutaraldéhyde est ensuite éliminé par 3 dialyses successives de 4 heures chacune effectuées dans un boudin de dialyse dont le seuil de coupure est de 3 kDa, à 4 °C, contre 200 ml de tampon phosphate pH 7,6 10 mM 150 mM NaCl.

Le mélange est alors traité par du formaldéhyde à la concentration finale de 33 mM pendant 48 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple comparatif 10 : Préparation d'un hétérocomplexe peptide Tat (19-50) - ₕIgE

Le peptide de Tat (19-50) apporte des épitopes auxiliaires helper.

Séquence du peptide Tat utilisé :

Lys-Thr-Ala-Cys-Thr-Asn-Cys-Tyr-Cys-Lys-Lys-Cys-Cys-Phe-His-
Cys-Gln-Val-Cys-Phe-Ile-Thr-Lys-Ala-Leu-Gly-Ile-Ser-Tyr-Gly-Arg-Lys

Ce conjugué représente le principe actif d'un immunogène propre à induire principalement chez le vacciné la formation d'anticorps anti-IgE (humaine).

0,1 mg de peptide Tat (19-50) (4,06 x 10⁻⁸ moles) sont dissous dans 0,2 ml de tampon borate 10 mM, pH 8,5. A cette solution sont ajoutés 1 mg d'IgE humaine (6,6 x 10⁻⁹ moles) dissous dans 1 ml du même tampon.

Le mélange ainsi constitué est traité par du glutaraldéhyde à la concentration finale de 0,026 M pendant 30 min. à la température ambiante.

L'excès de glutaraldéhyde est éliminé par 2 dialyses successives de 4 heures et par 1 dialyse finale de 16 heures contre un grand volume de tampon phosphate 10 mM, pH 7,4 contenant 0,8 % de NaCl (PBS), à 4°C, en utilisant un sac de dialyse avec un seuil de coupure à 10 kDa.
(rapport peptide IgE = 50:1)

### Exemple comparatif 11 : Préparation d'un hétérocomplexe contre le fragment de la Ricine

Cet hétérocomplexe constitue le principe actif d'un vaccin propre à induire chez le vacciné la formation d'anticorps neutralisants dirigés contre le fragment impliqué dans la fixation de la molécule de Ricine, l'empêchant ainsi d'exercer son activité toxique.

A 0,5 mg de KLH (1,1 x 10⁻³ moles) dissous dans 0,5 ml de tampon phosphate de 10 mM, pH 8,2 sont ajoutés 1 mg (3,3 x 10⁻⁸ moles) de fragment α de la Ricine dissous dans 1 ml du même tampon. Ce mélange est traité par le glutaraldéhyde à la concentration finale de 0,026 M pendant 30 min. à température ambiante.

L'excès de glutaraldéhyde est ensuite éliminé par dialyses successives de 4 heures chacune et par 1 dialyse de 16 heures contre un grand volume de tampon phosphate 10 mM, contenant 0,8 % de NaCl (PBS) en utilisant un sac de dialyse avec un seuil de coupure de 3 kDa.
(rapport KLH:Ricine-α = 1:30).

### Exemples de caractérisations biochimiques des hétérocomplexes

### A. Matériel et Méthodes des exemples 12 à 23.

Les caractérisations biochimiques des hétérocomplexes ont été effectuées à l'aide des techniques suivantes :

### 1.Test d'antigénicité :

L'étude de l'antigénicité d'un hétérocomplexe par rapport à l'antigénicité des protéines qui le composent est effectuée par un ELISA indirect classique. Cette technique permet de mesurer quantitativement une protéine par reconnaissance spécifique d'un anticorps contre un antigène. Ce test consiste à déposer des dilutions d'un échantillon contenant la protéine recherchée dans des puits d'une plaque de microtitration. Un anticorps polyclonal spécifique réagit avec la protéine immobilisée. Un deuxième anticorps, conjugué à la peroxydase de raifort, spécifique du premier est ensuite ajouté. Le complexe formé est révélé par incubation avec de l'OPD. La couleur jaune obtenue est directement proportionnelle à la quantité de protéines fixées. L'absorbance (DO) de chaque puits est mesurée à l'aide d'un lecteur de microplaques. La quantité de protéine présente dans l'échantillon est déterminée alors à l'aide d'une gamme d'étalonnage.

### 2. Isoélectrofocalisation en gel d'agarose suivie d'un Western Blot.

L'isoélectrofocalisation en gel d'agarose permet de séparer les molécules en fonction de leur point isoélectrique (pI) dans des conditions non dénaturantes, ce qui nous permet d'étudier les hétérocomplexes sans détruire les liaisons faibles existant au sein de ces complexes.

L'isoélectrofocalisation est suivie d'une révélation par Western blot. Après séparation électrophorétique, les molécules sont transférées sur une membrane de nitrocellulose par capillarité. Ces molécules sont ensuite caractérisées par immunochimie.

### 3. Mesure du pourcentage des molécules d'antigène d'intérêt liée à la molécule protéique porteuse par une liaison covalente (Premier procédé).

L'estimation du pourcentage des molécules d'antigène d'intérêt liées aux molécules protéiques porteuses par une liaison covalente dans un produit immunogène est effectuée par exemple par tamisage moléculaire sur une colonne contenant du Superdex 200, dans des conditions dénaturantes (urée 8 M) et réductrices (beta-mercaptoéthanol 5%).

Le % de molécules d'antigène d'intérêt liées par une liaison covalente est déduit de la quantité d'antigène d'intérêt (déterminée par un ELISA indirect classique) présente dans le volume d'exclusion de la colonne. En effet, la molécule protéique porteuse, telle le KLH, ayant un masse moléculaire plus important que la limite supérieure de fractionnement de la colonne utilisée (200 kDa dans notre cas), sort dans le volume d'exclusion de cette colonne. Ainsi, dans des conditions dénaturantes et réductrices, seuls les antigènes d'intérêt liés de manière covalente à la molécule protéique porteuse sortent dans le volume d'exclusion.

### 4. Mesure du pourcentage des molécules d'antigène d'intérêt liées à la molécule porteuse par une liaison covalente (Second procédé).

Le pourcentage de cytokine fixée sur la protéine porteuse (KLH) a été déterminé par un ELISA double sandwich, à l'aide d'un anticorps de capture dirigé spécifiquement contre la protéine porteuse.

100 µl d'anticorps polyclonaux de chevaux dirigés contre le KLH (1 mg/ml) dilués dans un tampon phosphate 10 mM pH 7,3 NaCl 150 mM (PBS) sont fixés dans des puits d'une plaque de microtitration (high-binding; Costar) pendant 2 heures à 37°C. Après 3 lavages dans du PBS/0.1% Tween 20 (PBST), les puits sont saturés avec du PBS contenant 2 % de SVF.

Après 1 h30 de saturation, les puits sont lavés 3 fois avec du PBST, puis des dilutions de 2 en 2 d'hétérocomplexe (10, 5, 2.5, 1.25, 0.625, 0.312 et 0.156 µg/ml), réalisées en doublon, sont ajoutées dans les puits (100 µl/puits).

Après 2 heures d'incubation, les puits sont lavés 3 fois avec le PBST. Le tween, agent dissociant, présent dans le tampon de lavage, permet d'éliminer toutes molécules non fixées de manière covalente sur le KLH qui lui est fixé spécifiquement sur l'anticorps de capture.

Ensuite les deux dilutions d'hétérocomplexe sont traitées de 2 façons différentes
a) la première série est incubée avec un anticorps dirigé contre le KLH
b) la deuxième série est incubée avec un anticorps dirigé contre la cytokine.

Après 1 h30 d'incubation à 37 °C, les puits sont lavés comme précédemment puis incubés avec un anticorps secondaire couplé à la peroxydase , dirigé contre l'espèce d'origine du premier anticorps. Après 1 h30 d'incubation à 37 °C, les puits sont à nouveau lavés. Ensuite l'addition du substrat de la peroxydase, O-PhénylèneDiamine, (OPD), permet de révéler la présence du KLH fixé par l'anticorps de capture et des cytokines fixées de manière covalente sur le KLH.

La quantité de KLH fixé par l'anticorps de capture puis la quantité de molécules de cytokine fixée sur le KLH de manière covalente sont calculées à l'aide de courbes d'étalonnage faites par ELISA.

Le pourcentage de cytokine fixée de manière covalente au KLH est alors déterminé.

### Exemple 12 : Caractérisation biochimique de l'hétérocomplexe KLH-VEGF murin

### 1.L'antigénicité

L'hétérocomplexe KLH-VEGF murin présente une antigénicité identique à l'antigénicité du VEGF murin.

### 2. Isoélectrofocalisation en gel d'agarose suivie d'un Western Blot.

La figure 1 montre que l'hétérocomplexe KLH-VEGF murin migre sous la forme d'une seule bande à un pI différent des molécules natives le constituant.

### Exemple 13 : Caractérisation biochimique de l'hétérocomplexe KLH-VEGF humain

### 1. Antigénicité :

L'hétérocomplexe KLH-VEGF humain présente une antigénicité identique à l'antigénicité du VEGF humain.

### 2. Isoélectrofocalisation suivie d'un Western Blot.

La figure 2 montre que l'hétérocomplexe KLH-VEGF humain migre sous la forme d'une seule bande à un pI différent des molécules natives le constituant. L'échantillon de VEGF humain a été déposé à trois endroits différents afin de montrer qu'il migre toujours au même endroit.

### Exemple 14 : Caractérisation biochimique de l'hétérocomplexe KLH-IL4murin

### 1. Antigénicité

L'hétérocomplexe KLH-IL4 murin présente une antigénicité identique à l'antigénicité de l'IL4 murin.

### Exemple 15 : Caractérisation biochimique de l'hétérocomplexe KLH-IL4 humain

### 1. Antigénicité

Le complexe KLH-IL4 humain présente une antigénicité égale à l'antigénicité de la protéine IL4 humain.

### 2. Isoélectrofocalisation suivie d'un Western Blot.

La figure 3 montre que l'hétérocomplexe KLH-IL4 humain migre sous la forme d'une seule bande à un pI différent des molécules natives le constituant.

### Exemple 16 : Caractérisation biochimique de l'hétérocomplexe KLH-IFNα

### 1. Antigénicité

Le complexe KLH-IFNα humain présente une antigénicité identique à l'antigénicité de I'IFNα humain.

### 2. Estimation du porcentage des molécules d'antigène d'intérêt liées à la molécule protéique porteuse par une liaison covalente.

La préparation KLH-IFNα a été passée sur une colonne superdex S200 selon les conditions décrites plus haut. Le pic sorti dans le volume d'exclusion a été collecté, dialysé puis lyophilisé. La concentration en antigène d'intérêt a été déterminée par la technique d'ELISA indirecte. La quantité d'IFNα retrouvée dans le volume exclu est de 30 µg alors que 1000 µg d'IFN ont été utilisés pour préparer le produit immunogène, sans perte mesurable d'antigène durant le procédé de préparation. Le pourcentage de molécules d'antigène d'intérêt liées à la molécule KLH par une liaison covalente dans le produit immunogène comprenant des hétérocomplexes KLH-IFNα peut donc être estimé à environ 3 %

### Exemple 17 Caractérisation biochimique de l'hétérocomplexe gp160-IFNα

### 1. Antigénicité

Le complexe gp160-IFNα humain présente une antigénicité identique à l'antigénicité de la protéine gp160 ainsi qu'à celle de l'IFNα humain.

### 2. Isoélectrofocalisation suivie d'un Western Blot.

La figure 4 montre que l'hétérocomplexe gp160-IFNαhumain migre sous la forme d'une seule bande à un pI qui est très différent du pl de la protéine gp160 recombinante le constituant. Le pl de cet hétérocomplexe est légèrement inférieur à celui de l'IFNα.

### Exemple comparatif 18 : Caractérisation biochimique de l'hétérocomplexe gp160-Tat toxoide

### 1. Antigénicité

Le complexe gp160-Tat Toxoid présente une antigénicité identique à l'antigénicité de la protéine gp160 et à celle de la protéine Tat.

### Exemple comparatif 19 : Caractérisation biochimique de l'hétérocomplexe gp160-Tat GM

### 1-Antigénicité

Le complexe gp160-Tat GM présente une antigénicité identique à l'antigénicité de la protéine gp160 et à celle de la protéine Tat.

### Exemple 20 : Caractérisation biochimique de l'hétérocomplexe KLH-IL4murin

### 1. Antigénicité

L'hétérocomplexe KLH-IL4 murin présente une antigénicité identique à l'antigénicité de l'IL4 murin.

### 2. Estimation du % de molécules d'antigènes d'intérêts fixées de manière covalente à la molécule protéique porteuse.

11 % de molécules d'IL4 murin sont fixées de manière covalente au KLH.

### Exemple 21 : Caractérisation biochimique de l'hétérocomplexe KLH-IFNα

### 1. Antigénicité

Le complexe KLH-IFNα humain présente une antigénicité identique à l'antigénicité de l'IFNα humain.

### 2. Estimation du % de molécules d'antigènes d'intérêts fixées de manière covalente à la molécule protéique porteuse.

8 % de molécules d'IFNα humain sont fixées de manière covalente au KLH.

### Exemple comparatif 22 : Caractérisation biochimique de l'hétrocomplexe peptide Tat-ₕIgE

### 1. Antigénicité

Le complexe peptide Tat-ₕIgE présente une antigénicité comparable à celle de l'IgE humaine.

### Exemple comparatif 23 : Caractérisation biochimique de l'hétérocornplexe KLH-Ricine-β

### 1. Antigénicité

Le complexe KLH-Ricine-β présente une antigénicité comparable à celle du fragment β de la Ricine.

### 2. Isoélectrofocalisation suivie d'un Western Blot

Le complexe migre sous la forme d'une bande unique et la présence du fragment β est mise en évidence par Western Blot.

### Exemples d'activité immunogénique des hétérocomplexes

### Exemple 24 : Activité immunogénique de l'hétérocomplexe KLH-VEGF murin

### A. Matériel et méthodes

L'activité immunogénique (humorale) de la préparation KLH-VEGF murin par rapport à celle du VEGF murin a été étudiée chez la souris balb c de 18-20 g.

### 1-immunisation

Au jour 0, 7, 14, 21 un groupe de 8 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (50 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 1. Absence de toxicité de l'hétérocomplexe in vivo et in vitro

Les souris immunisées aussi bien par la préparation de KLH-VEGF murin que par le VEGF murin uniquement ne présentent aucun signe clinique et aucune lésion anatomique. Le test d'immunosuppression montre que des doses de 100 ng/ml à 1 µg/ml de KLH-VEGF murin ne diminuent pas la prolifération des lymphocytes.

Aucune des 3 souris immunisées avec 50 µg de l'hétérocomplexe ne manifeste de signes de toxicité (température, troubles cutanés, manifestations systémiques ou régionales) pendant les 7 jours suivant l'injection.

### 2-Réponse humorale

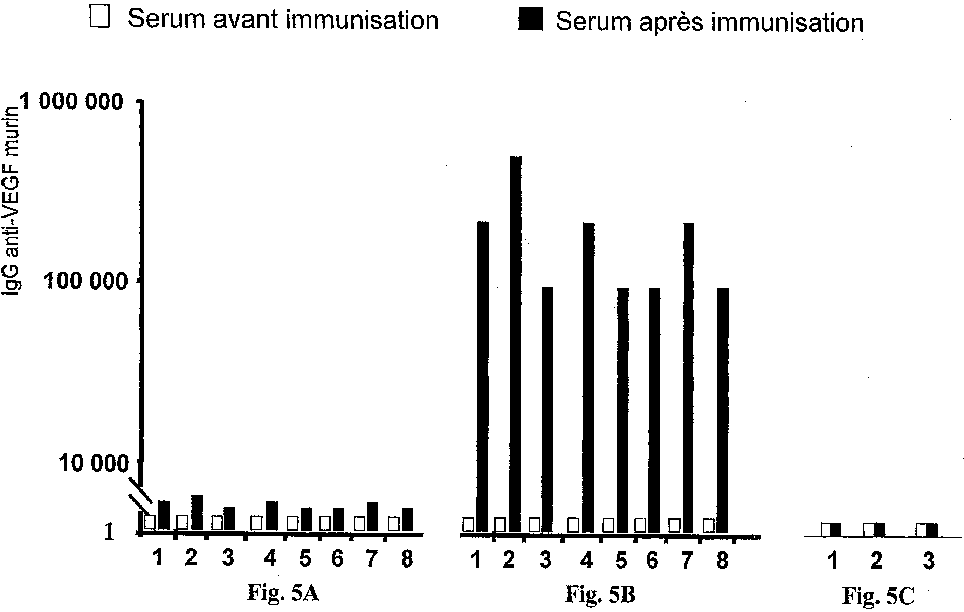

La réponse humorale est mesurée par la présence dans le sérum d'anticorps de type IgG dirigés contre le VEGF murin, déterminée par ELISA et exprimée en titre (inverse de la dilution donnant une densité optique supérieure à 0.3). La figure 5 représente les titres en anticorps obtenus.

Les souris immunisées avec la préparation KLH-VEGF murin présentent des titres d'anticorps de type IgG plus importants que ceux des souris immunisées avec le VEGF murin uniquement.

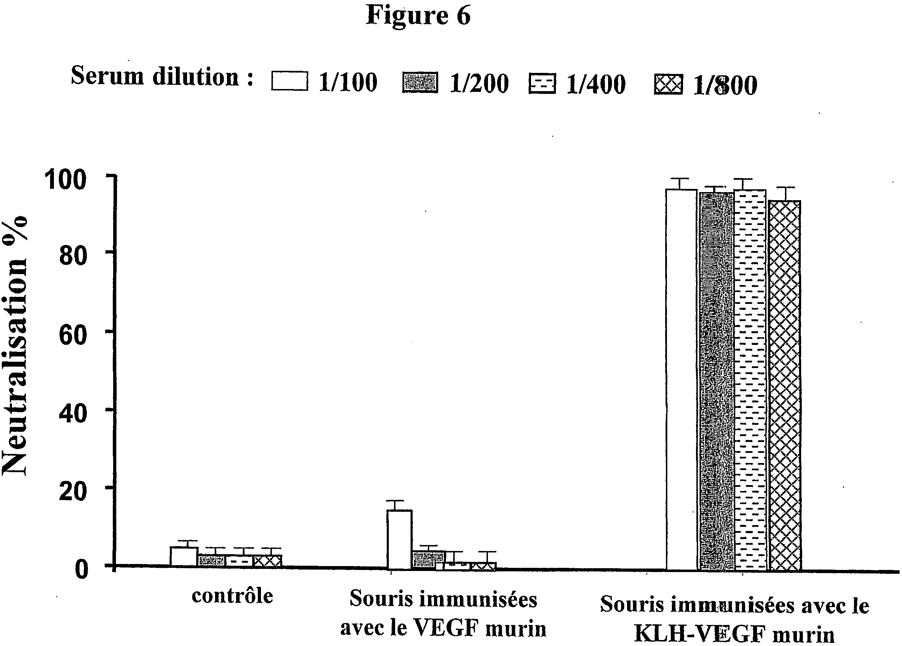

L'activité neutralisante de ces anticorps a été mesurée à l'aide du test d'activité biologique du VEGF, facteur de croissance sélectif des cellules endothéliales. Des cellules endothéliales (HUVECs) sont cultivées dans des puits à fond plat d'une plaque de microculture à raison de 3000 cellules par puits. Les sérums de chaque groupe de souris ont été regroupés. Différentes dilutions de ces pools de sérums (1/100 - 1/800) prélevés à J-2 et J72 ont été pré-incubées pendant 2 heures avec 20 ng/ml de VEGF murin puis déposées sur ces cellules endothéliales. La culture cellulaire s'est poursuivie à 37 °C en atmosphère humide chargée à 5 % de CO2 pendant 3 jours. 18 heures avant la fin de l'incubation, 0.5 µCi de thymidine tritiée/puits sont ajoutés. Les sérums neutralisants empêchent le VEGF murin d'induire la prolifération des cellules endothéliales, alors que les sérums non neutralisants permettent la prolifération de ces cellules. Les résultats sont donnés en pourcentage de neutralisation. La figure 6 présente les résultats obtenus.

Les anticorps induits par le complexe ont un pouvoir neutralisant plus important que celui induit par le VEGF murin.

### Exemple 25 : Activité immunogénique de l'hétérocomplexe KLH-VEGF humain

### A. Matériel et méthodes

L'activité immunogénique (humorale) de la préparation KLH-VEGF humain par rapport à celle du VEGF humain a été étudiée chez la souris balb c de 18-20 g.

### 1-immunisation

Au jour 0, 7, 14, 21 un groupe de 8 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (50 µg) selon la pharmacopée.

L'absence d'immunotoxicité de l'hétérocomplexe est évaluée in vitro par un test de prolifération cellulaires réalisé sur des PBMCs cultivées en présence du complexe et stimulées par du PPD ou du Tétanos toxoïde.

### B. Résultats :

### 1-Absence de toxicité de l'hétérocomplexe in vivo et in vitro

Les souris immunisées aussi bien par la préparation de KLH-VEGF humain que par le VEGF humain uniquement ne présentent aucun signe clinique et aucune lésion anatomique. Le test d'immunosuppression montre que des doses de 100 ng/ml à 1 µg/ml de KLH-VEGF humain ne diminuent pas la prolifération des lymphocytes.

Aucune des 3 souris immunisées avec 50 µg de l'hétérocomplexe ne manifeste de signes de toxicité (température, troubles cutanés, manifestations systémiques ou régionales) pendant les 7 jours suivant l'injection.

### 2-Réponse humorale

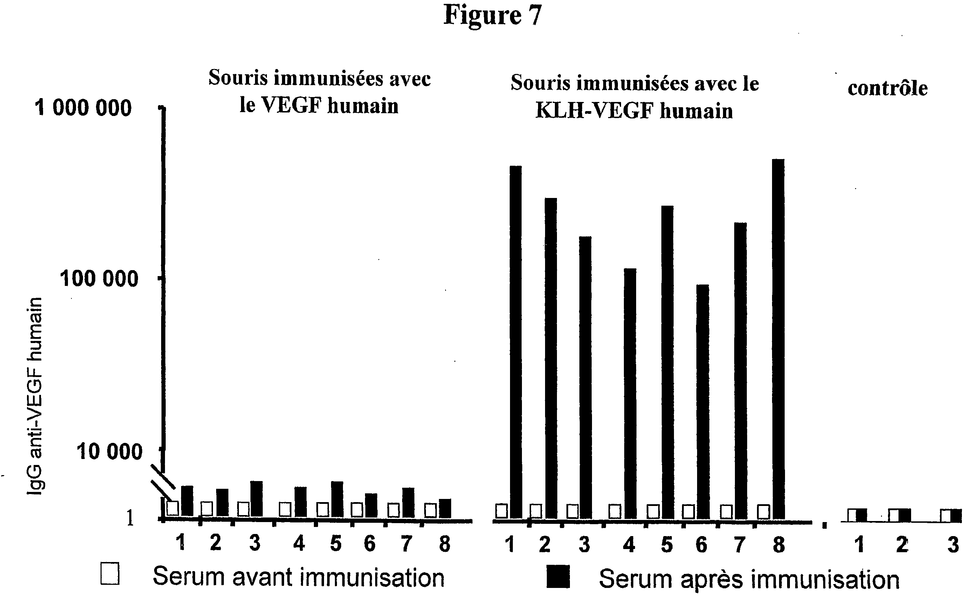

La réponse humorale est mesurée par la présence dans le sérum d'anticorps de type IgG dirigés contre le VEGF humain, déterminée par ELISA et exprimée en titre (inverse de la dilution donnant une densité optique supérieure à 0.3). La figure 7 représente les titres en anticorps obtenus.

Les souris immunisées avec la préparation KLH-VEGF humain présentent des titres d'anticorps de type IgG plus importants que ceux des souris immunisées avec le VEGF humain uniquement.

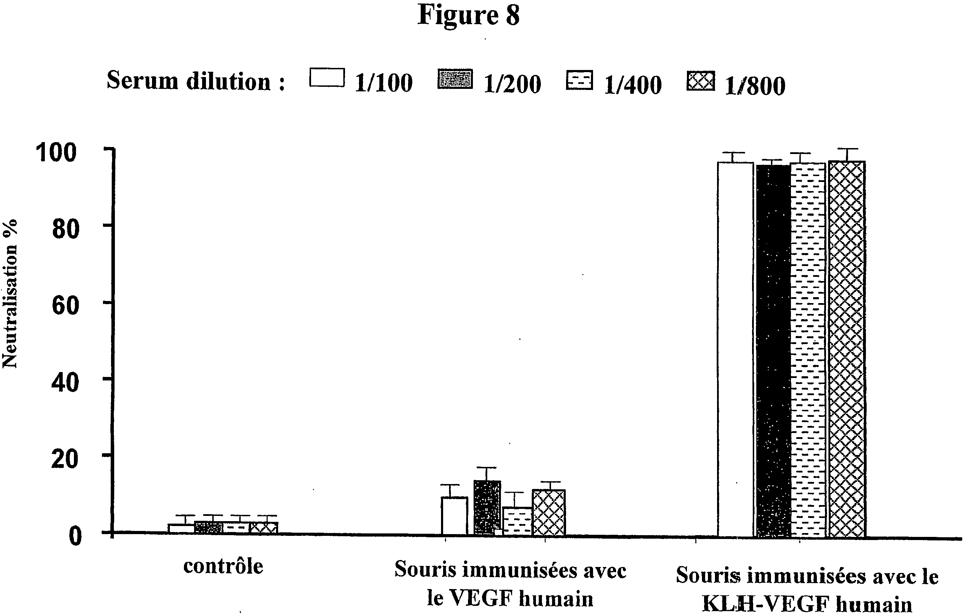

L'activité neutralisante de ces anticorps a été mesurée à l'aide du test d'activité biologique du VEGF, facteur de croissance sélectif des cellules endothéliales. Des cellules endothéliales (HUVECs) sont cultivées dans des puits à fond plat d'une plaque de microculture à raison de 3000 cellules par puits. Les sérums de chaque groupe de souris ont été regroupés. Différentes dilutions de ces pools de sérums (1/100 - 1/800) prélevés à J-2 et J72 sont pré-incubées pendant 2 heures avec 20 ng/ml de VEGF humain puis déposées sur ces cellules endothéliales. La culture cellulaire est poursuivie à 37 °C en atmosphère humide chargée à 5 % de CO2 pendant 3 jours. 18 heures avant la fin de l'incubation, 0.5 µCi de thymidine tritiée/puits sont ajoutés. Les sérums neutralisants empêchent le VEGF humain d'induire la prolifération des cellules endothéliales, alors que les sérums non neutralisants permettent la prolifération de ces cellules. Les résultats sont donnés en pourcentage de neutralisation. La figure 8 présente les résultats obtenus.

Les anticorps induits par le complexe ont un pouvoir neutralisant plus important que celui induit par le VEGF humain.

### Exemple 26 : Activité immunogénique de l'hétérocomplexe KLH-IL4 murin

### A. Matériel et méthodes

L'activité immunogénique (humorale) de la préparation KLH-IL4 murin par rapport à celle de l'IL4 murin a été étudiée chez la souris balb c de 18-20 g.

### 1-immunisation

Au jour 0, 7, 14, 21 un groupe de 8 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2 à J72
3 souris contrôles reçoivent les mêmes préparations sans immunogène.
14 jours après la dernière immunisation, les souris contrôles et les souris immunisées avec le KLH-IL4 murin sont challengées avec du pollen de bouleau en présence d'alun (100 µg/souris) en sous-cutanée à J74, J95 et J109. Des prélèvements sanguins sont effectués régulièrement pour suivre l'apparition des anticorps de classe G et E dirigés contre le Bet v 1, allergène majeur du pollen de bouleau.

### 2-toxicité

La toxicité anormale est recherchée chez 3 souris recevant 1 dose humaine (50 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 1. Absence de toxicité de l'hétérocomplexe in vivo et in vitro

Les souris immunisées aussi bien par la préparation de KLH-IL4 murin que par l'IL4 murin uniquement ne présentent aucun signe clinique et aucune lésion anatomique. Le test d'immunosuppression montre que des doses de 100 ng/ml à 1 µg/ml de KLH-IL4 murin ne diminuent pas la prolifération des lymphocytes.

Aucune des 3 souris immunisées avec 50 µg de l'hétérocomplexe ne manifeste de signes de toxicité (température, troubles cutanés, manifestations systémiques ou régionales) pendant les 7 jours suivant l'injection.

### 2-Réponse humorale

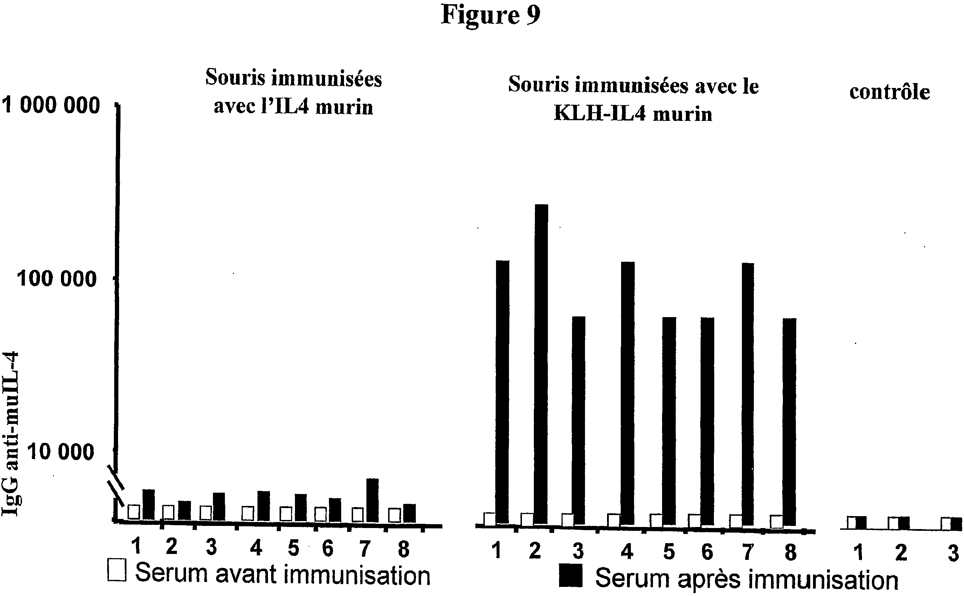

La réponse humorale est mesurée par la présence dans le sérum d'anticorps de type IgG dirigés contre l'IL4 murin, déterminée par ELISA et exprimée en titre (inverse de la dilution donnant une densité optique supérieure à 0.3). La figure 9 représente les titres en anticorps obtenus.

Les souris immunisées avec la préparation KLH-IL4 murin présentent des titres d'anticorps de type IgG plus importants que ceux des souris immunisées avec le IL4 murin uniquement.

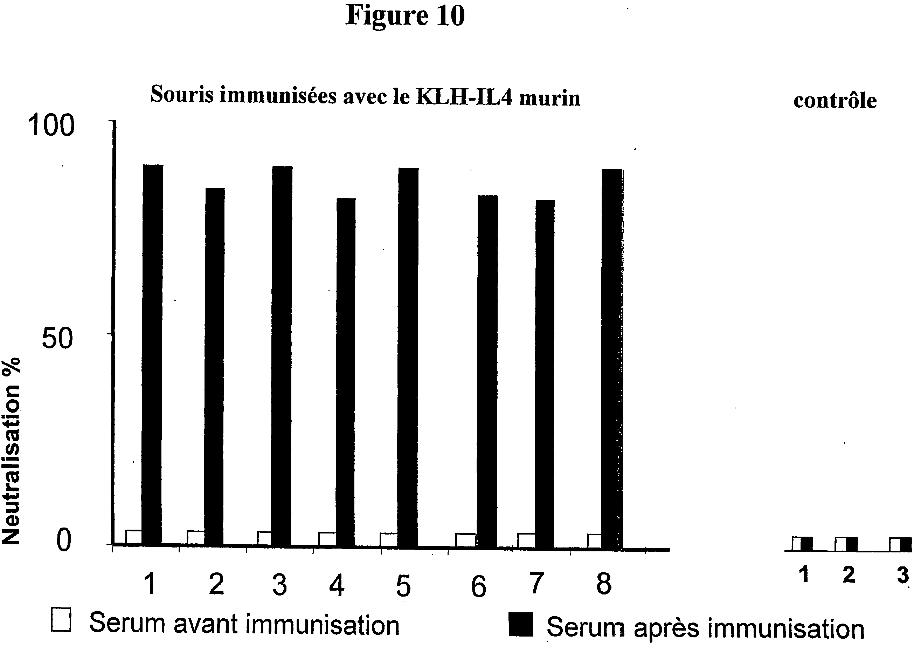

L'activité neutralisante de ces anticorps présents chez les souris immunisées avec la préparation KLH-IL4 murin a été mesurée à l'aide du test d'activité biologique de l'IL4 murin. Ce test utilise les cellules HT-2, lignées cellulaires murines dont la croissance est II4 murine -dépendante (Watson, J. 1979. J. Exp. Med. 150:1510.). Des cellules HT-2 sont cultivées dans des puits à fond rond d'une plaque de microculture à raison de 10 000 cellules par puits. Des sérums dilués au 1/50 prélevés à J-2 et J72 sont pré-incubés pendant 2 heures avec 50 ng/ml d'II4 murin puis déposés sur les cellules HT-2 . La culture cellulaire est poursuivie à 37 °C en atmosphère humide chargée à 5 % de CO2 pendant 3 jours. 4 heures avant la fin de l'incubation, 0.5 µCi de thymidine tritiée/puits sont ajoutés. Les sérums neutralisants empêchent l'IL4 murin d'induire la prolifération des cellules HT-2, alors que les sérums non neutralisants permettent la prolifération de ces cellules. Les résultats sont donnés en pourcentage de neutralisation. La figure 10 présente les résultats obtenus.

Les anticorps induits par le complexe sont neutralisants.

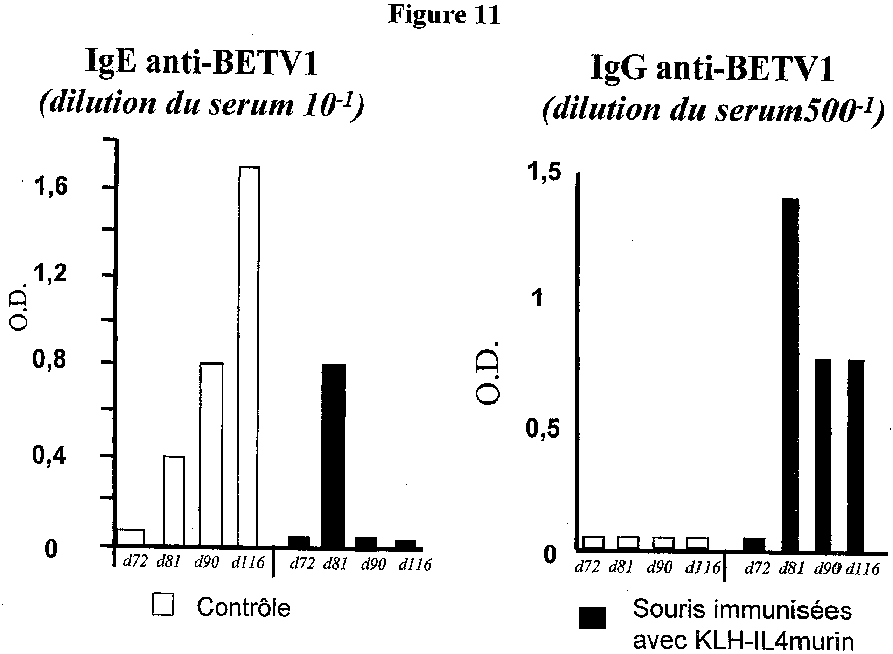

De plus, ces anticorps neutralisants dirigés contre l'II4 murin empêchent la production, par ces souris, d'anticorps de type IgE dirigés contre le Bet v 1, lorsque ces dernières sont challengées avec du pollen de bouleau. La figure 11 montre bien que les souris immunisées avec du KLH-IL4murin possédant des IgG neutralisants dirigés contre l'IL4 murin bloquent la production d'IgE dirigés contre le Bet v 1 et commencent à produire des anticorps de type IgG dirigés contre le Bet v 1. Par contre, les souris qui n'ont pas reçu de KLH-IL4 murin, et qui n'ont donc pas d'anticorps de type IgG dirigés contre l'IL4, produisent uniquement des anticorps de type IgE dirigés contre le Bet v 1.

### Exemple 27 : Activité immunogénique de l'hétérocomplexe KLH-IL4 humain

### A. Matériel et méthodes

L'activité immunogénique (humorale) de la préparation KLH-IL4 humain par rapport à celle de l'IL4 humain a été étudiée chez la souris balb c de 18-20 g.

### 1-immunisation

Au jour 0, 7, 14, 21 un groupe de 3 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (50 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 1-Absence de toxicité de l'hétérocomplexe in vivo et in vitro

Les souris immunisées aussi bien par la préparation de KLH-IL4 humain que par l'IL4 humain uniquement ne présentent aucun signe clinique et aucune lésion anatomique. Le test d'immunosuppression montre que des doses de 100 ng/ml à 1 µg/ml de KLH-IL4 humain ne diminuent pas la prolifération des lymphocytes.

Aucune des 3 souris immunisées avec 50 µg de l'hétérocomplexe ne manifeste de signes de toxicité (température, troubles cutanés, manifestations systémiques ou régionales) pendant les 7 jours suivant l'injection.

### 2-Réponse humorale

La réponse humorale est mesurée par la présence dans le sérum d'anticorps de type IgG dirigés contre l'IL4 humain, déterminée par ELISA et exprimée en titre (inverse de la dilution donnant une densité optique supérieure à 0.3).

**Tableau 1**

| Titre | | |
|---|---|---|
| j-2 | j72 | |
| *souris contrôles :* | | |
| souris contrôle 1 | <500⁻¹ | <500⁻¹ |
| souris contrôle 2 | <500⁻¹ | <500⁻¹ |
| souris contrôle 3 | <500⁻¹ | <500⁻¹ |
| | | |

| *souris immunisées avec l'IL4 humain :* | | |
|---|---|---|
| souris 4 | <500⁻¹ | 32 000⁻¹ |
| souris 5 | <500⁻¹ | 48 000⁻¹ |
| souris 6 | <500⁻¹ | 16 000⁻¹ |

| *souris immunisées avec le complexe KLH-IL4 humain :* | | |
|---|---|---|
| souris 7 | <500⁻¹ | 256 000⁻¹ |
| souris 8 | <500⁻¹ | 128 000⁻¹ |
| souris 9 | <500⁻¹ | 128 000⁻¹ |

Les souris immunisées avec la préparation KLH-IL4 humain présentent des titres d'anticorps de type IgG plus importants que ceux des souris immunisées avec le IL4 humain uniquement.

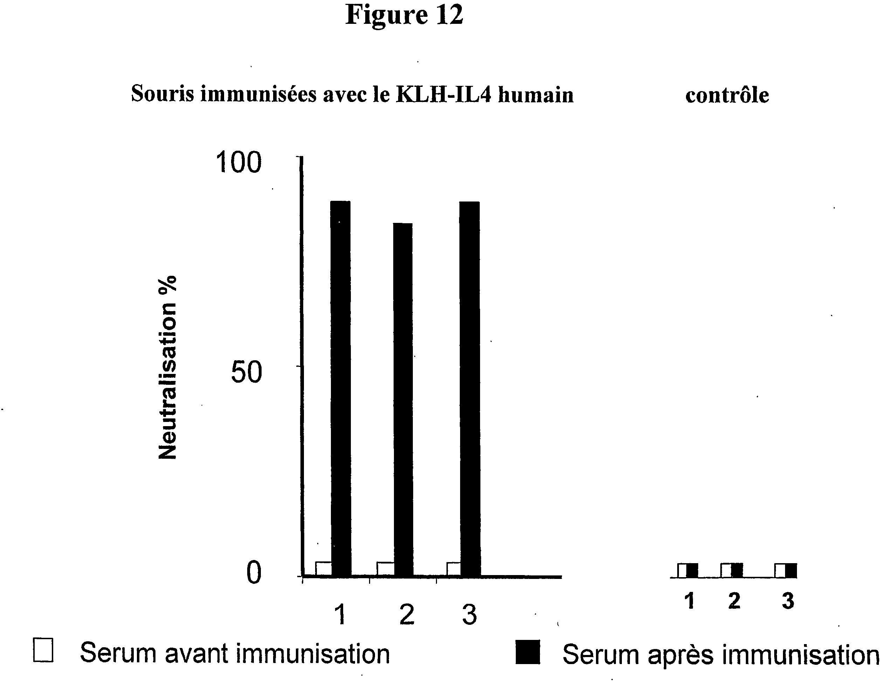

L'activité neutralisante de ces anticorps induits par la préparation KLH-IL4 humain a été mesurée à l'aide du test d'activité biologique de l'IL4 humain. Ce test utilise les cellules TF-1, lignée cellulaire humaine dont la croissance est IL4 humaine-dépendante (Kitamura, T. et al., 1989.. J. Cell Physiol. 140:323-34). Des cellules TF-1 sont cultivées dans des puits à fond rond d'une plaque dé microculture à raison de 10 000 cellules par puits. Des sérums dilués au 1/50 prélevés à J-2 et J72 pré-incubés pendant 2 heures avec 50 ng/ml d'IL4 humain sont ensuite déposés sur les cellules TF-1 . La culture cellulaire est poursuivie à 37 °C en atmosphère humide chargée à 5 % de CO2 pendant 3 jours. 4 heures avant la fin de l'incubation, 0.5 µCi de thymidine tritiée/puits sont ajoutés. Les sérums neutralisants empêchent l'IL4 humain d'induire la prolifération des cellules TF-1, alors que les sérums non neutralisants permettent la prolifération de ces cellules. Les résultats sont donnés en pourcentage de neutralisation. La figure 12 présente les résultats obtenus.

Les anticorps induits par le complexe sont neutralisants.

### Exemple 28 : Activité immunogénique de l'hétérocomplexe KLH-IFNα

### A. Matériel et méthodes

L'activité immunogénique (humorale) de la préparation KLH-IFNα humain par rapport à celle de l'IFNα humaine a été étudiée chez la souris balb c de 18-20 g.

### 1-immunisation

Au jour 0, 7, 14, 21 un groupe de 3 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (100 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 1-Absence de toxicité de l'hétérocomplexe in vivo et in vitro

Les souris immunisées aussi bien par la préparation de KLH-IFNα humain que par l'IFNα humain uniquement ne présentent aucun signe clinique et aucune lésion anatomique. Le test d'immunosuppression montre que des doses de 100 ng/ml à 1 µg/ml de KLH-IFNα humain ne diminuent pas la prolifération des lymphocytes.

Aucune des 3 souris immunisées avec 100 µg de l'hétérocomplexe ne manifeste de signes de toxicité (température, troubles cutanés, manifestations systémiques ou régionales) pendant les 7 jours suivant l'injection.

### 2-Réponse humorale

La réponse humorale est mesurée par la présence dans le sérum d'anticorps de type IgG dirigés contre l'IFNα humain, déterminée par ELISA et exprimée en titre (inverse de la dilution donnant une densité optique supérieure à 0.3). Le tableau 2 représente les titres en anticorps obtenus.

**TABLEAU 2**

| | | Titre |
|---|---|---|
| | j-2 | j72 |
| *souris contrôles :* | | |
| souris contrôle 1 | <500⁻¹ | <500⁻¹ |
| souris contrôle 2 | <500⁻¹ | <500⁻¹ |
| souris contrôle 3 | <500⁻¹ | <500⁻¹ |

| *souris immunisées avec l'IFN*α : | | |
|---|---|---|
| souris 4 | <500⁻¹ | 96 000⁻¹ |
| souris 5 | <500⁻¹ | 128 000⁻¹ |
| souris 6 | <500⁻¹ | 96 000⁻¹ |

| *souris immunisées avec le complexe KLH-IFN*α : | | |
|---|---|---|
| souris 7 | <500⁻¹ | 96 000⁻¹ |
| souris 8 | <500⁻¹ | 96 000⁻¹ |
| souris 9 | <500⁻¹ | 128 000⁻¹ |

Les souris immunisées avec la préparation KLH-IFNα humain présentent des titres d'anticorps de type IgG équivalents à ceux des souris immunisées avec l'IFNα humain uniquement.

L'activité neutralisante de ces anticorps a été mesurée à l'aide du test d'activité biologique de l'IFNα humain. (Rubinstein S, J Virol, 1981,755-8). Ce test de mesure d'effet antiviral a pour but d'évaluer l'inhibition de la lyse de cellules MDBK par le VSV (Vesicular Stomatitis virus) en présence d'IFN. Des cellules MDBK sont cultivées dans des puits à fond rond d'une plaque de microculture à raison de 350 000 cellules par puits. Différentes dilutions de sérums (1/100 au 1/800) prélevés à J-2 et J72 sont pré-incubées pendant 2 heures avec 5 ng/ml d'IFNα humain puis déposées sur les cellules MDBK . Après 20 heures de culture cellulaire réalisée à 37°C en atmosphère humide chargée à 5 % de CO2, les sérums dilués présents dans les puits sont enlevés, les cellules lavées, puis 100 µl contenant 100 DL50 (dose léthale 50 %) de virus VSV sont ajoutés. 18 heures après l'addition du virus, l'effet lytique du virus est mesuré. Les sérums neutralisants permettent au VSV de lyser les cellules, alors que les sérums non neutralisants empêchent cette lyse. Les résultats sont donnés en pourcentage de neutralisation.

**TABLEAU 3**

| *souris immunisées avec l'IFN*α : | | | | |
|---|---|---|---|---|
| | 1/100 | 1/200 | 1/400 | 1/800 |
| souris 4 j-2 | 0 | 0 | 0 | 0 |
| j72 | 100 | 75 | 65 | 50 |
| souris 5 j-2 | 0 | 0 | 0 | 0 |
| j72 | 100 | 67 | 60 | 55 |
| souris 6 j-2 | 0 | 0 | 0 | 0 |
| j72 | 100 | 72 | 65 | 60 |

| *souris immunisées avec le conjugué KLH-IFN*α *:* | | | | |
|---|---|---|---|---|
| | 1/100 | 1/200 | 1/400 | 1/800 |
| souris 7 j-2 | 0 | 0 | 0 | 0 |
| j72 | 100 | 100 | 100 | 100 |
| souris 8 j-2 | 0 | 0 | 0 | 0 |
| j72 | 100 | 100 | 100 | 100 |
| souris 9 j-2 | 0 | 0 | 0 | 0 |
| j72 | 100 | 100 | 100 | 100 |

Les anticorps induits par le complexe ont un pouvoir neutralisant plus important que celui induit par l'IFNα humain. Les résultats sont donnés en % de neutralisation.

### Exemple 29 : Activité immunogénique de l'hétérocomplexe gp160-IFNα

### A. Matériel et méthodes

L'activité immunogénique (humorale) de la préparation gp160-IFNα humain par rapport à celle de l'IFNα humaine a été étudiée chez la souris balb c de 18-20 g.

### 1-immunisation

Au jour 0, 7, 14, 21 un groupe de 3 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (100 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 1-Absence de toxicité de l'hétérocomplexe in vivo et in vitro

Les souris immunisées aussi bien par la préparation de gp160-IFN humain que par l'IFNα humain uniquement ne présentent aucun signe clinique et aucune lésion anatomique. Le test d'immunosuppression montre que des doses de 100 ng/ml à 1 µg/ml de gp160-IFNα humain ne diminuent pas la prolifération des lymphocytes.

Aucune des 3 souris immunisées avec 100 µg de l'hétérocomplexe ne manifeste de signes de toxicité (température, troubles cutanés, manifestations systémiques ou régionales) pendant les 7 jours suivant l'injection.

### 2-Réponse humorale

La réponse humorale est mesurée par la présence dans le sérum d'anticorps de type IgG dirigés contre l'IFN humain, déterminée par ELISA et exprimée en titre (inverse de la dilution donnant une densité optique supérieure à 0.3).

**TABLEAU 4**

| | | Titre |
|---|---|---|
| | j-2 | j72 |
| *souris contrôles :* | | |
| souris contrôle 1 | <500⁻¹ | <500⁻¹ |
| souris contrôle 2 | <500⁻¹ | <500⁻¹ |
| souris contrôle 3 | <500⁻¹ | <500⁻¹ |
| | | |

| *souris immunisées avec l'IFN*α : | | |
|---|---|---|
| souris 4 | <500⁻¹ | 64 000⁻¹ |
| souris 5 | <500⁻¹ | 96 000⁻¹ |
| souris 6 | <500⁻¹ | 128 000⁻¹ |
| | | |

| *souris immunisées avec le complexe gp160-IFN*α : | | |
|---|---|---|
| souris 7 | <500⁻¹ | 96 000⁻¹ |
| souris 8 | <500⁻¹ | 96 000⁻¹ |
| souris 9 | <500⁻¹ | 64 000⁻¹ |

Les souris immunisées avec la préparation gp160-IFNα humain présentent des titres d'anticorps de type IgG équivalents à ceux des souris immunisées avec l'IFNα humain uniquement.

L'activité neutralisante de ces anticorps a été mesurée à l'aide du test d'activité biologique de l'IFNα humain décrit dans l'exemple précédant. Les résultats sont données en % de neutralisation.

**TABLEAU 5**

| *souris immunisées avec l'IFN*α : | | | | |
|---|---|---|---|---|
| | 1/100 | 1/200 | 1/400 | 1/800 |
| souris 4 j-2 | 0 | 0 | 0 | 0 |
| j72 | 100 | 80 | 70 | 53 |
| souris 5 j-2 | 0 | 0 | 0 | 0 |
| j72 | 100 | 70 | 65 | 50 |
| souris 6 j-2 | 0 | 0 | 0 | 0 |
| j72 | 100 | 65 | 60 | 57 |

| *souris immunisées avec le conjugué gp160-IFN*α : | | | | |
|---|---|---|---|---|
| | 1/100 | 1/200 | 1/400 | 1/800 |
| souris 7 j-2 | 0 | 0 | 0 | 0 |
| j72 | 100 | 100 | 100 | 100 |
| souris 8 j-2 | 0 | 0 | 0 | 0 |
| j72 | 100 | 100 | 100 | 100 |
| souris 9 j-2 | 0 | 0 | 0 | 0 |
| j72 | 100 | 100 | 100 | 100 |

Les anticorps induits par le complexe ont un pouvoir neutralisant plus important que celui induit par l'IFNα humain.

### Exemple comparatif 30 : Activité immunogénigue de l'hétérocomplexe gp160-Tat toxoide

### A. Matériel et méthodes

L'activité immunogénique (humorale et cellulaire) de la préparation gp160-Tat toxoide par rapport à celle du Tat toxoide a été étudiée chez la souris balb c de 18-20 g.

### 1-immunisation

Au jour 0, 7, 14, 21 un groupe de 3 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (100 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 1-Absence de toxicité de l'hétérocomplexe in vivo et in vitro

Les souris immunisées aussi bien par la préparation de gp160-Tat toxoide que par le Tat toxoide uniquement ne présentent aucun signe clinique et aucune lésion anatomique. Le test d'immunosuppression montre que des doses de 100 ng/ml à 1 µg/ml de gp160-Tat toxoide ne diminuent pas la prolifération des lymphocytes.

Aucune des 3 souris immunisées avec 100 µg de l'hétérocomplexe ne manifeste de signes de toxicité (température, troubles cutanés, manifestations systémiques ou régionales) pendant les 7 jours suivant l'injection.

### 2-Réponse humorale

La réponse humorale est mesurée par la présence dans le sérum d'anticorps de type IgG dirigés contre le Tat, déterminée par ELISA et exprimée en titre (inverse de la dilution donnant une densité optique supérieure à 0.3). Le tableau 1 représente les titres en anticorps obtenus.

**TABLEAU 6**

| | | Titre |
|---|---|---|
| | j-2 | J72 |
| *souris contrôles :* | | |
| souris contrôle 1 | <500⁻¹ | <500⁻¹ |
| souris contrôle 2 | <500⁻¹ | <500⁻¹ |
| souris contrôle 3 | <500⁻¹ | <500⁻¹ |

| *souris immunisées avec le Tat toxoïde :* | | |
|---|---|---|
| souris 4 | <500⁻¹ | 48 000⁻¹ |
| souris 5 | <500⁻¹ | 64 000⁻¹ |
| souris 6 | <500⁻¹ | 48 000⁻¹ |

| *souris immunisées avec le conjugué gp160-Tat toxoïde :* | | |
|---|---|---|
| souris 7 | <500⁻¹ | 64 000⁻¹ |
| souris 8 | <500⁻¹ | 128 000⁻¹ |
| souris 9 | <500⁻¹ | 64 000⁻¹ |

Les souris immunisées avec la préparation de gp160-Tat toxoïde présentent des titres d'anticorps de type IgG anti-Tat plus importants que ceux des souris immunisées avec le Tat toxoïde uniquement.

L'activité neutralisante de ces anticorps a été mesurée à l'aide du Cat assay. Différentes dilutions de sérums (1/100- 1/800) prélevés à J-2 et J72 sont incubées pendant 2 heures avec 50 ng/ml de Tat natif. Ces dilutions sont ensuite déposées sur des cellules HeLa, cellules transfectées de manière stable avec un plasmide contenant le LTR du VIH-1 comme promoteur du gène Chloramphénicol Acétyl transférase (CAT). Après 24 heures de culture, les cellules sont lysées et la quantité de protéine CAT produite est mesurée par un test ELISA, le Cat assay, (Boehringer Mannheim). Les sérums neutralisants empêchent la protéine Tat d'induire l'expression de la protéine CAT, alors que les sérums non neutralisants permettent la synthèse de cette protéine CAT.Les résultats sont données en % de neutralisation.

**TABLEAU 7**

| *souris immunisées avec le Tat toxoïde :* | | | | |
|---|---|---|---|---|
| | 1/100 | 1/200 | 1/400 | 1/800 |
| souris 4 j-2 | 0 | 0 | 0 | 0 |
| j72 | 60 | 50 | 25 | 20 |
| souris 5 j-2 | 0 | 0 | 0 | 0 |
| j72 | 60 | 55 | 30 | 20 |
| souris 6 j-2 | 0 | 0 | 0 | 0 |
| j72 | 65 | 50 | 30 | 30 |

| *souris immunisées avec le conjugué gp160-Tat toxoïde :* | | | | |
|---|---|---|---|---|
| | 1/100 | 1/200 | 1/400 | 1/800 |
| souris 7 j-2 | 0 | 0 | 0 | 0 |
| j72 | 100 | 100 | 100 | 100 |
| souris 8 j-2 | 0 | 0 | 0 | 0 |
| j72 | 100 | 100 | 100 | 100 |
| souris 9 j-2 | 0 | 0 | 0 | 0 |
| j72 | 100 | 100 | 100 | 100 |

Les anticorps induits par le conjugué gp160-Tat toxoïde ont un pouvoir neutralisant plus important que celui induit par le Tat toxoïde.

### 2. Production de MIP1α

La production de MIP1α dans les surnageants de culture des splénocytes. Les splénocytes des souris immunisées et des souris contrôles sont isolées puis cultivées dans des puits à fond rond d'une plaque de micro-culture à raison de 100 000 cellules/puits en présence de 5 µg/ml de p24, de gp160, de Tat natif et d'un mélange de 5 µg/ml gp160 et de 5 µg/ml de Tat natif. Les surnageants sont prélevés après 24 heures de culture et la présence de MIP1 dans les surnageants est mesurée à l'aide d'un test ELISA de R&D. Les résultats sont exprimés en pg/ml.

**TABLEAU 8**

| | Gp160 | | Tat nati | gp160 + Tat natif | | p24 |
|---|---|---|---|---|---|---|
| *Souris contrôles:* | | | | | | |
| Souris 1 | j72 | MIP1α | 95 | 90 | 145 | 9 |
| Souris 2 | j72 | MIP1α | 100 | 90 | 136 | 7 |
| Souris 3 | j72 | MIP1α | 120 | 110 | 132 | 9 |

| *Souris immunisées par le Tat toxoïde:* | | | | | | |
|---|---|---|---|---|---|---|
| Souris 4 | j72 | MIP1α | 145 | 130 | 190 | 7 |
| Souris 5 | j72 | MIP1α | 128 | 145 | 225 | 9 |
| Souris 6 | j72 | MIP1α | 150 | 230 | 295 | 10 |

| *Souris immunisées par le conjugué gp160-Tat toxoïde* | | | | | | |
|---|---|---|---|---|---|---|
| Souris 7 | j72 | MIP1α | 875 | 736 | 1725 | 9 |
| Souris 8 | j72 | MIP1α | 945 | 905 | 1900 | 7 |
| Souris 9 | j72 | MIP1α | 1025 | 795 | 1755 | 8 |

Les splénocytes des souris immunisées avec le conjugué gp160-Tat toxoïde produisent plus de chimiokines MIP1α que les cellules des souris immunisées par le Tat toxoïde uniquement lorsqu'elles sont activées, *in vitro,* par les immunogènes utilisés lors de l'immunisation.

### 4. Prolifération des splénocytes de souris immunisées (test CMI)

Les splénocytes des souris immunisées et des souris contrôles sont isolées puis cultivées dans des puits à fond rond d'une plaque de micro-culture à raison de 100 000 cellules/puits en présence de p24, de gp160, de Tat natif et d'un mélange de gp160 et de Tat natif. La culture cellulaire est poursuivie à 37 °C en atmosphère humide chargée à 5 % de CO2 pendant 6 jours. 18 heures avant la fin de l'incubation, 0.5 µCi de thymidine tritiée/ puits sont ajoutés. L'intensité de la réponse immunitaire est proportionnelle à l'index de prolifération Ip.
Ip = cpm (coups par minute) pour l'antigène donné / cpm contrôle

**TABLEAU 9**

| | | Gp160 | Tat natif | gp160 + Tat natif | p24 |
|---|---|---|---|---|---|
| *Souris contrôles :* | | | | | |
| Souris 1 | j72 | 1,1 | 1,1 | 1 | 1,2 |
| Souris 2 | j72 | 1 | 1,1 | 1,1 | 1,1 |
| Souris 3 | j72 | 1,2 | 1 | 1 | 1,1 |

| *Souris immunisées avec le Tat toxoïde* | | | | | |
|---|---|---|---|---|---|
| Souris 4 | j72 | 1,2 | 8 | 10 | 1,1 |
| Souris 5 | j72 | 1 | 9 | 9 | 1,2 |
| Souris 6 | j72 | 1 | 10 | 9 | 1,2 |

| *Souris immunisées avec le conjugué gp160-Tat toxoïde :* | | | | | |
|---|---|---|---|---|---|
| Souris 7 | j72 | 9 | 11 | 8 | 1 |
| Souris 8 | j72 | 10 | 9 | 7,5 | 1 |
| Souris 9 | j72 | 10,5 | 9 | 8 | 1 |

Les splénocytes des souris immunisées avec le conjugué gp160-Tat toxoïde ou le Tat toxoïde, prolifèrent, lorsqu'elles sont activées, in vitro, avec les immunogènes utilisés lors de l'immunisation.

### Exemple comparatif 31 : Activité immunogénique de l'hétérocomplexe gp160-Tat GM

### A. Matériel et méthodes

L'activité immunogénique (humorale et cellulaire) de la préparation gp160-Tat GM par rapport à celle du Tat toxoide a été étudiée chez la souris balb c de 18-20 g.

### 1-immunisation

Au jour 0, 7, 14, 21 un groupe de 3 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (100 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 1-Absence de toxicité de l'hétérocomplexe in vivo et in vitro

Les souris immunisées aussi bien par la préparation de gp160-Tat GM que par le Tat toxoide uniquement ne présentent aucun signe clinique et aucune lésion anatomique. Le test d'immunosuppression montre que des doses de 100 ng/ml à 1 µg/ml de gp160-Tat toxoide ne diminuent pas la prolifération des lymphocytes.

Aucune des 3 souris immunisées avec 100 µg de l'hétérocomplexe ne manifeste de signes de toxicité (température, troubles cutanés, manifestations systémiques ou régionales) pendant les 7 jours suivant l'injection.

### 2-Réponse humorale

La réponse humorale est mesurée par la présence dans le sérum d'anticorps de type IgG dirigés contre le Tat, déterminée par ELISA et exprimée en titre (inverse de la dilution donnant une densité optique supérieure à 0.3). Le tableau 1 représente les titres en anticorps obtenus.

**TABLEAU 10**

| | Titre | |
|---|---|---|
| *souris contrôles :* | j-2 | j72 |
| souris contrôle 1 | <500⁻¹ | <500⁻¹ |
| souris contrôle 2 | <500⁻¹ | <500⁻¹ |
| souris contrôle 3 | <500⁻¹ | <500⁻¹ |
| | | |

| *souris immunisées avec le Tat GM :* | | |
|---|---|---|
| souris 4 | <500⁻¹ | 64 000⁻¹ |
| souris 5 | <500⁻¹ | 64 000⁻¹ |
| souris 6 | <500⁻¹ | 48 000⁻¹ |

| *souris immunisées avec le conjugué gp160-Tat GM :* | | |
|---|---|---|
| souris 7 | <500⁻¹ | 128 000⁻¹ |
| souris 8 | <500⁻¹ | 128 000⁻¹ |
| souris 9 | <500⁻¹ | 64 000⁻¹ |

Les souris immunisées avec la préparation de gp160-Tat GM présentent des titres d'anticorps de type IgG anti-Tat plus importants que ceux des souris immunisées avec le Tat GM uniquement.

### Exemple 32 : Activité immunogénique de l'hétérocomplexe KLH-TNFα murin

### Matériel et méthodes

L'activité immunogénique (humorale) de la préparation KLH-TNFα murin par rapport à celle du TNFα murin a été étudiée chez la souris Balb/c de 18-20 g.
Au jour 0, un groupe de 3 souris (groupe A) reçoit une injection de 0.1 ml d'une émulsion en AIF par voie intramusculaire contentant 60 µg de l'hétérocomplexe KLH-TNFα. Une injection de rappel de 30 µg et de 15 µg en AIF est donnée respectivement à J21 et J60. 3 souris contrôles reçoivent une dose équivalente en TNFα murin selon le même protocole. (groupe B)
Au jour 0, un groupe de 3 souris (groupe C) reçoit une injection de 0.1 ml d'une émulsion en AlF par voie intramusculaire contentant 60 µg de l'hétérocomplexe KLH-TNFα murin et 30 µg de l'oligodeoxynucleotide phosphorothioate 5'-TCCATGACGTTCCTGACGTT-3' (CpG DNA: 1826). Une injection de rappel de 30 µg et de 15 µg d'hétérocomplexe KLH-TNFα murin en AIF est donnée respectivement à J21 et J60. 3 souris contrôles reçoivent une dose équivalente en TNFα murin selon le même protocole. (groupe D)
Un prélèvement sanguin au niveau rétro-orbital est effectué sur chaque souris avant la première injection à j-2
Les souris sont sacrifiées 12 jours après la dernière immunisation.

### 2-toxicité

La toxicité anormale est recherchée chez 3 souris recevant 1 dose humaine (50 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 1- Absence de toxicité de l'hétérocomplexe in vivo et in vitro

Les souris immunisées aussi bien par la préparation de KLH-TNFα murin que par le TNFα murin uniquement ne présentent aucun signe clinique et aucune lésion anatomique. Le test d'immunosuppression montre que des doses de 100 ng/ml à 1 µg/ml de KLH-TNFα murin ne diminuent pas la prolifération des lymphocytes.

Aucune des 3 souris immunisées avec 50 µg de l'hétérocomplexe avec ou sans le CpG DNA 1826 ne manifeste de signes de toxicité (température, troubles cutanés, manifestations systémiques ou régionales) pendant les 7 jours suivant l'injection.

### 2-Réponse humorale

La réponse humorale est mesurée par la présence dans le sérum d'anticorps de type IgG dirigés contre le TNFα murin, déterminée par ELISA et exprimée en titre. La présence d'anticorps de type IgA dirigés contre le TNFα murin dans les sécrétions vaginales a également été déterminée par ELISA et exprimée en titre. Le titre représente l'inverse de la dilution donnant une densité optique supérieure à 0.3. Le tableau suivant représente les titres en anticorps obtenus.

**TABLEAU 11**

| | | | IgA vaginale | |
|---|---|---|---|---|
| | J-2 | j72 | J-2 | j72 |
| Souris immunisées avec KLH-TNFa murin (groupe A) | | | | |
| 1 | < 500⁻¹ | 64 000⁻¹ | < 10⁻¹ | 20⁻¹ |
| 2 | | 48 000⁻¹ | | 20⁻¹ |
| 3 | | 64 000⁻¹ | | 40⁻¹ |

| Souris Immunisées avec le TNFa murin (groupe B) | | | | |
|---|---|---|---|---|
| 4 | < 500⁻¹ | 750⁻¹ | < 10⁻¹ | 10⁻¹ |
| 5 | | 1 000⁻¹ | | 20⁻¹ |
| 6 | | 750⁻¹ | | 10⁻¹ |

| Souris immunisées avec le KLH-TNFa murin en présence de CpG (groupe C) | | | | |
|---|---|---|---|---|
| 7 | < 500⁻¹ | 128 000⁻¹ | < 10⁻¹ | 160⁻¹ |
| 8 | | 256 000⁻¹ | | 80⁻¹ |
| 9 | | 256 000⁻¹ | | 320⁻¹ |

| Souris Immunisées avec le TNFa murin en présence de CpG (groupe D) | | | | |
|---|---|---|---|---|
| 7 | < 500⁻¹ | 2 000⁻¹ | < 10⁻¹ | 20⁻¹ |
| 8 | | 4 000⁻¹ | | 40⁻¹ |
| 9 | | 3 000⁻¹ | | 40⁻¹ |

### Exemple comparatif 33 : Activité immunogénique de l'hétérocomplexe de peptide de Tat-ₕIgE

### A. Matériel et méthodes

L'activité immunogénique (humorale) de la préparation KLH-IgE humaine par rapport à celle de l'IgE humaine a été étudiée chez la souris Balb/c de 18-20 g.

### 1-immunisation

Au jour 0, 7, 14, 21 un groupe de 3 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (50 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 2- Absence de toxicité de l'hétérocomplexe in vivo et in vitro

Les souris immunisées aussi bien par la préparation de KLH-IgE humaine que par l'IgE humaine uniquement ne présentent aucun signe clinique et aucune lésion anatomique. Le test d'immunosuppression montre que des doses de 100 ng/ml à 1 µg/ml de KLH-IgE humaine ne diminuent pas la prolifération des lymphocytes.

Aucune des 3 souris immunisées avec 50 µg de l'hétérocomplexe ne manifeste de signes de toxicité (température, troubles cutanés, manifestations systémiques ou régionales) pendant les 7 jours suivant l'injection.

### 2-Réponse humorale

La réponse humorale est mesurée par la présence dans le sérum d'anticorps de type IgG dirigés contre l'IgE humaine, déterminée par ELISA et exprimée en titre (inverse de la dilution donnant une densité optique supérieure à 0.3. Le tableau suivant représente les titres en anticorps obtenus.

**TABLEAU 12**

| | Titre | |
|---|---|---|
| | **J-2** | **j72** |
| **Souris contrôles** | | |
| **1** | < **500⁻¹** | < **500⁻¹** |
| **2** | | |
| **3** | | |
| | | |

| **Souris immunisées avec ₕIgE** | | |
|---|---|---|
| **4** | < **500⁻¹** | **64 000⁻¹** |
| **5** | | **128 000⁻¹** |
| **6** | | **128 000⁻¹** |
| | | |

| Souris immunisées avec KLH-ₕIgE | | |
|---|---|---|
| **7** | < **500⁻¹** | **256 000⁻¹** |
| **8** | | **128 000⁻¹** |
| **9** | | **256 000⁻¹** |

Les souris immunisées avec la préparation KLH-ₕIgE présentent des titres d'anticorps de type IgG légèrement supérieurs à ceux de souris immunisées avec la préparation de ₕIgE.

### Exemple comparatif 34 : Activité immunogénique de l'hétérocomplexe KLH-Ricine-β

### B. Matériel et méthodes

L'activité immunogénique (humorale) de la préparation KLH-Ricine-β par rapport à celle du fragment β de la Ricine a été étudiée chez la souris Balb/c de 18-20 g.

### 1-immunisation

Au jour 0, 7, 14, 21 un groupe de 3 souris reçoit une injection de 0.1 ml (10 µg) d'une émulsion en AIF par voie intramusculaire. Une injection de rappel de 5 µg en AIF est donnée à J60.

Un prélèvement sanguin au niveau rétro-orbital est effectué sur chaque souris avant la première injection à j-2
3 souris contrôles reçoivent les mêmes préparations sans immunogène.

Les souris sont sacrifiées 12 jours après la dernière immunisation.

### 2-toxicité

La toxicité anormale est recherchée chez 3 souris recevant 1 dose humaine (50 µg) selon la pharmacopée.

L'absence d'immunotoxicité de l'hétérocomplexe est évaluée in vitro par un test de prolifération cellulaire réalisé sur des PBMCs cultivées en présence du complexe et stimulées par du PPD ou du Tétanos toxoïde.

### B. Résultats :

### 3- Absence de toxicité de l'hétérocomplexe in vivo et in vitro

Les souris immunisées aussi bien par la préparation de KLH-Ricine- humaine que par le fragment β de la Ricine uniquement ne présentent aucun signe clinique et aucune lésion anatomique. Le test d'immunosuppression montre que des doses de 100 ng/ml à 1 µg/ml de KLH-Ricine-β ne diminuent pas la prolifération des lymphocytes.

Aucune des 3 souris immunisées avec 50 µg de l'hétérocomplexe ne manifeste de signes de toxicité (température, troubles cutanés, manifestations systémiques ou régionales) pendant les 7 jours suivant l'injection.

### 2-Réponse humorale

La réponse humorale est mesurée par la présence dans le sérum d'anticorps de type IgG dirigés contre le fragment β de la Ricine, déterminée par ELISA et exprimée en titre (inverse de la dilution donnant une densité optique supérieure à 0.3. Le tableau suivant représente les titres en anticorps obtenus.

**TABLEAU 13**

| | Titre | |
|---|---|---|
| | **J-2** | **j72** |
| **Souris contrôles** | | |
| **1** | < **500⁻¹** | < **500⁻¹** |
| **2** | | |
| **3** | | |
| | | |

| **Souris immunisées avec Ricine-β** | | |
|---|---|---|
| **4** | < **500⁻¹** | **256 000⁻¹** |
| **5** | | **512 000⁻¹** |
| **6** | | **256 000⁻¹** |
| | | |

| Souris immunisées avec KLH-Ricine-β | | |
|---|---|---|
| **7** | < **500⁻¹** | **256 000⁻¹** |
| **8** | | **256 000⁻¹** |
| **9** | | **128 000⁻¹** |

L'activité neutralisante de ces anticorps a été vérifiée par l'injection à la souris de mélanges de sérum anti-Ricine-β et de Ricine qui n'ont pas entraîné la mort de l'animal, à l'inverse de ce qui a été observé lors de l'administration à la souris de mélanges de Ricine et de sérum normal.

### REFERENCES

Adler HL, McCurdy MA, Kattan MW, Timme TL, Scardino PT, Thompson TC. Elevated levels of circulating interleukin-6 and transforming growth factor-beta1 in patients with metastatic prostatic carcinoma. J Urol 1999;161:182-7
Aucouturier J et al, Adjuvants designed for veterinary and human vaccines. Vaccine 2001 19 :2666-72
Baras B. et al, Single-dose mucosal immunization with biodegradable microparticles containing a Schistosoma mansoni antigen. Infect Immun. (1999) 67:2643-8)
Basak A, Boudreault A, Chen A, Chretien M, Seidah NG, Lazure C., Application of the multiple antigenic peptides (MAP) strategy to the production of prohormone convertases antibodies: synthesis, characterization and use of 8-branched immunogenic peptides. : J Pept Sci 1995 Nov-Dec;1 (6):385-95
Cowan et al, Induction of TNF alpha in human neuronal cells by extracellular human T-cell lymphotropic virus Type 1 Tax1, Journal of virology, 1997, 6982-6989. ,
Ferreira FK et al, 1993, Purification and characterization of recombinant Bet v1, the major Birch pollen allergen : immunological equivalence to natural Bet v1; J. Biol. Chem., 268 : 19574.
Fouts TR, Tuskan R, Godfrey K, Reitz M, Hone D, Lewis GK, DeVico AL. Expression and characterization of a single-chain polypeptide analogue of the human immunodeficiency virus type 1 gp120-CD4 receptor complex. J. Virol. 2000 Dec., 74(24):11427-36.
Fouts T. Godfrey K, Bobb K, Montefiori D, Hanson CV, Kalyanaraman VS, DeVico A, Pal R. Cross-linked HIV-1 envelop-CD4 receptor complexes elicit broadly cross-reactive neutralizing antibodies in rhesus macaques. Proc. Natl. Acad. Sci. USA 2002, September 3; 99(18):11842-7. Epun 2002 Aug. 21.
Le Buanec et al, HPV-16 E7 but not E6 oncogenic protein triggers both cellular immunosuppression and angiogenic processes. Biomed Pharmacother. 1999;53: 424-31.
Morgenstern JP et al, Amino acid sequence of Feld1, the major allergen of the domestic cat : protein sequence analysis and cDNA cloning., PNAS, 1991, 88 : 9690
Mori N et al, Interleukine-10 gene expression in adult t-cell leukaemia, Blood, 1996, 1035-45.
Sementchenko VI, Schweinfest CW, Papas TS, Watson DK., ETS2 function is required to maintain the transformed state of human prostate cancer cells. Oncogene 1998;17:2883-8
Tovey ER et al, Mite faeces are a major source of house dust allergens. Nature, 1981. 289 : 592-593.
Yoshiji H et al, Expression of vascular endothelial growth factor, its receptor, and other angiogenic factors in human breast cancer. Cancer Res 1996, 56 :2013-6
Zagury D et al, Interferon alpha and Tat involvement in the immunosuppression of uninfected T cells and C-C chemokine decline in AIDS. Proc Natl Acad Sci U S A 1998;95 : 3851-6
Zusman I, Sandler B, Gurevich P, Zusman R, Smirnoff P, Tendler Y, Bass D, Shani A, Idelevich E, Pfefferman R, Davidovich B, Huszar M, Glick J. Comparative study of the role of serum levels of p53 antigen and its tumor cell concentration in colon cancer detection. Hum Antibodies Hybridomas. (1996)

## Revendications

1. Produit immunogène stable pour l'induction d'anticorps à l'encontre d'une ou plusieurs protéines antigéniques chez un sujet, **caractérisé en ce qu'**il consiste en des hétérocomplexes immunogènes protéiques constitués d'associations entre (i) des molécules de protéines antigéniques et (ii) des molécules protéiques porteuses, **en ce que** au moins 1 pour cent et **en ce que** moins de 40 pour cent des protéines antigéniques (i) sont liées avec les molécules protéiques porteuses (ii) par une liaison covalente, et **en ce que** la ou les protéines antigéniques (i) consistent en des cytokines produites naturellement par ledit sujet.

2. Produit immunogène selon la revendication 1, **caractérisé en ce que** chaque hétérocomplexe comprend (i) une pluralité de protéines antigéniques liées à (ii) une molécule protéique porteuse.

3. Produit immunogène selon la revendication 2, **caractérisé en ce que**, pour chaque hétérocomplexe immunogène, la pluralité de protéines antigéniques (i) est constituée d'une pluralité d'exemplaires d'une protéine antigénique unique.

4. Produit immunogène selon l'une des revendications 2 ou 3, **caractérisé en ce que**, pour chaque hétérocomplexe immunogène, les protéines antigéniques (i) sont constituées d'une pluralité d'exemplaires d'une protéine normalement reconnue comme une protéine du soi par les cellules du système immunitaire dudit sujet.

5. Produit selon l'une des revendications 1 à 4, **caractérisé en ce qu'**il comprend de 5 à 50 protéines antigéniques (i) pour une molécule protéique porteuse (ii), de préférence de 20 à 40 protéines antigéniques (i) pour une molécule protéique porteuse ii).

6. Produit immunogène selon l'une des revendications 1 à 5, **caractérisé en ce que** les liaisons covalentes entre une ou plusieurs des protéines antigéniques (i) et la molécule protéique porteuse (ii) sont réalisées par l'intermédiaire d'un agent chimique de liaison bifonctionnel.

7. Produit immunogène selon la revendication 6, **caractérisé en ce que** ledit agent chimique de liaison comprend au moins deux fonctions aldéhydes libres.

8. Produit immunogène selon la revendication 7, **caractérisé en ce que** ledit agent de liaison est le glutaraldéhyde.

9. Produit immunogène selon l'une des revendications 1 à 8, **caractérisé en ce que** la ou les protéines antigéniques (i) sont choisies parmi l'interleukine-4, l'interféron alpha, l'interféron gamma, le VEGF, l'interleukine-10, le TNF alpha, le TGF béta, l'interleukine-5 et l'interleukine 6.

10. Produit immunogène selon l'une des revendications 1 à 9, **caractérisé en ce qu'**il est choisi parmi les produits comprenant les hétéro complexes suivants, dans lesquels les protéines antigéniques (i), d'une part et la molécule porteuse protéique (ii), d'autre part, sont respectivement
a) (i) IL-4 et (ii) KLH ;
b) (i) interféron alpha et (ii) KLH ;
c) (i) VEGF et (ii) KLH ;
d) (i) IL-10 et (ii) KLH ;
e) (i) interféron alpha et (ii) gp164 de VIH1
f) (i) IL-4 et (ii) l'antigène allergène Bet v1 ;
g) (i) le VEGF et (ii) la protéine E7 d'un Papillomavirus, et
h) (i) le TNFα et (ii) KLH.

11. Composition comprenant un produit immunogène selon l'une des revendications 1 à 10.

12. Composition pharmaceutique comprenant un produit immunogène selon l'une des revendications 1 à 10, en association avec un ou plusieurs excipients physiologiquement compatibles.

13. Composition immunogène comprenant un produit immunogène selon l'une des revendications 1 à 10, en association avec un ou plusieurs excipients physiologiquement compatibles.

14. Composition vaccinale comprenant un produit immunogène selon l'une des revendications 1 à 10, en association avec un ou plusieurs excipients physiologiquement compatibles.

15. Composition immunogène ou composition vaccinale selon l'une des revendications 13 ou 14, **caractérisée en ce qu'**elle comprend l'adjuvant de l'immunité CpG.

16. Procédé de préparation d'un produit immunogène selon l'une des revendications 1 à 9, **caractérisé en ce qu'**il comprend les étapes suivantes :
a) incuber les protéines antigéniques (i) consistant en des cytokines produites naturellement par un sujet, et la molécule porteuse (ii) dans un rapport molaire (i) : (ii) de 5 : 1 à 50 : 1, en présence d'un agent chimique de liaison;
b) stabiliser les hétérocomplexes formés à l'étape a) par traitement avec le formadéhyde, et
c) récupérer le produit immunogène comprenant des hétérocomplexes immunogènes qui est préparé à l'étape b).

17. Procédé selon la revendication 16, **caractérisé en ce que** l'agent chimique de liaison est le glutaraldéhyde.

## Patentansprüche

1. Stabiles immunogenes Produkt zur Induzierung von Antikörpern gegen ein oder mehrere antigene Proteine in einem Subjekt, **dadurch gekennzeichnet, dass** es aus immunogenen Proteinheterokomplexen besteht, die aus Assoziationen von (i) antigenen Proteinmolekülen und (ii) Trägerproteinmolekülen aufgebaut sind, wobei mindestens 1 % und weniger als 40 % der antigenen Proteine (i) mit den Trägerproteinmolekülen (ii) mittels einer kovalenten Bindung verbunden sind, und dass das oder die antigenen Proteine (i) aus Cytokinen bestehen, die natürlicherweise von dem Subjekt hergestellt werden.

2. Immunogenes Produkt nach Anspruch 1, **dadurch gekennzeichnet, dass** jeder Heterokomplex (i) eine Vielzahl von antigenen Proteinen, die an ein Trägerproteinmolekül (ii) gebunden sind, umfasst.

3. Immunogenes Produkt nach Anspruch 2, **dadurch gekennzeichnet, dass** für jeden immunogenen Heterokomplex die Vielzahl der antigenen Proteine (i) aus einer Vielzahl von Exemplaren eines einzigen antigenen Proteins aufgebaut ist.

4. Immunogenes Produkt nach einem der Ansprüche 2 oder 3, **dadurch gekennzeichnet, dass** für jeden immunogenen Heterokomplex die antigenen Proteine (i) aus einer Vielzahl von Exemplaren eines Proteins aufgebaut sind, das normalerweise von den Zellen des Immunsystem des Subjekts als Selbstprotein erkannt wird.

5. Produkt nach einem der Ansprüche 1 bis 4, **dadurch gekennzeichnet, dass** es 5 bis 50 antigene Proteine (i) für ein Trägerproteinmolekül (ii) umfasst, bevorzugt 20 bis 40 antigene Proteine (i) für ein Trägerproteinmolekül (ii).

6. Immunogenes Produkt nach einem der Ansprüche 1 bis 5, **dadurch gekennzeichnet, dass** die kovalenten Bindungen zwischen einem oder mehreren der antigenen Proteine (i) und dem Trägerproteinmolekül (ii) mittels eines bifunktionellen chemischen Bindemittels realisiert werden.

7. Immunogenes Produkt nach Anspruch 6, **dadurch gekennzeichnet, dass** das chemische Bindungsmittel mindestens zwei freie Aldehydfunktionen aufweist.

8. Immunogenes Produkt nach Anspruch 7, **dadurch gekennzeichnet, dass** das Bindungsmittel Glutaraldehyd ist.

9. Immunogenes Produkt nach einem der Ansprüche 1 bis 8, **dadurch gekennzeichnet, dass** das oder die antigenen Proteine (i) aus Interleukin-4, Interferon-alpha, Interferongamma, VEGF, Interleukin-10, TNF-alpha, TGF-beta, Interleukin-5 und Interleukin-6 ausgewählt sind.

10. Immunogenes Produkt nach einem der Ansprüche 1 bis 9, **dadurch gekennzeichnet, dass** es aus Produkten ausgewählt ist, welche die folgenden Heterokomplexe umfassen, worin die antigenen Proteine (i) einerseits und das Trägerproteinmolekül (ii) andererseits jeweils sind:
a) (i) IL-4 und (ii) KLH;
b) (i) Interferon-alpha und (ii) KLH;
c) (i) VEGF und (ii) KLH;
d) (i) IL-10 und (ii) KLH;
e) (i)Interferon-alpha und (ii) gp 160 von VIH1
f) (i) IL-4 und (ii) allergenes Antigen Bet v 1
g) (i) VEGF und (ii) das Papillomaprotein E7, und
h) (i)TNFα und (ii) KLH.

11. Zusammensetzung, welche ein immunogenes Produkt nach einem der Ansprüche 1 bis 10 umfasst.

12. Pharmazeutische Zusammensetzung, umfassend ein immunogenes Produkt nach einem der Ansprüche 1 bis 10 zusammen mit einem oder mehreren physiologisch verträglichen Exzipienten.

13. Immunogene Zusammensetzung, umfassend ein immunogenes Produkt nach einem der Ansprüche 1 bis 10 zusammen mit einem oder mehreren physiologisch verträglichen Exzipienten.

14. Impfzusammensetzung, umfassend ein immunogenes Produkt nach einem der Ansprüche 1 bis 10 zusammen mit einem oder mehreren physiologisch verträglichen Exzipienten.

15. Immunogene Zusammensetzung oder Impfzusammensetzung nach einem der Ansprüche 13 oder 14, **dadurch gekennzeichnet, dass** die Zusammensetzung das Immunitätsadjuvans CpG umfasst.

16. Verfahren zur Herstellung eines immunogenen Produkts nach einem der Ansprüche 1 bis 9, **dadurch gekennzeichnet, dass** es die folgenden Schritte aufweist:
a) Inkubieren der antigenen Proteine (i), bestehend aus Cytokinen, die natürlicherweise von einem Subjekt hergestellt werden, und des Trägermoleküls (ii) in einem Molverhältnis (i):(ii) von 5:1 bis 50:1 in Gegenwart eines chemischen Bindungsmittels;
b) Stabilisieren der in Schritt (a) gebildeten Heterokomplexe durch Behandlung mit Formaldehyd und
c) Gewinnen des in Schritt b) gebildeten immunogenen Produkts, welches die immunogenen Heterokomplexe umfasst.

17. Verfahren nach Anspruch 16, **dadurch gekennzeichnet, dass** das chemische Bindungsmittel Glutaraldehyd ist.

## Claims

1. Stable immunogenic product for the induction of antibodies against one or more antigenic proteins in a subject, **characterized in that** it consists of immunogenic protein heterocomplexes consisting of associations between (i) molecules of antigenic proteins and (ii) carrier protein molecules, and **in that** at least 1 per cent and less than 40 per cent of the antigenic proteins (i) are linked to the carrier protein molecules (ii) through a covalent bond, and that the antigenic protein or proteins (i) consist of cytokines produced naturally by the said subject.

2. Immunogenic product according to claim 1, **characterized in that** each heterocomplex comprises (i) a plurality of antigenic proteins linked to (ii) a carrier protein molecule.

3. Immunogenic product according to claim 2, **characterized in that**, for each immunogenic heterocomplex, the plurality of antigenic proteins (i) consists of a plurality of a single antigenic protein.

4. Immunogenic product according to any one of claims 2 or 3, **characterized in that**, for each immunogenic heterocomplex, the antigenic proteins (i) consist of a plurality of a protein normally recognised as a 'self' protein by the cells of the immune system of the said subject.

5. Product according to any one of claims 1 to 4, **characterized in that** it comprises from 5 to 50 antigenic proteins (i) for one carrier protein molecule (ii), and preferably from 20 to 40 antigenic proteins (i) for one carrier protein molecule (ii).

6. Immunogenic product according to any one of claims 1 to 5, **characterized in that** the covalent links between one or more of the antigenic proteins (i) and the carrier protein molecule (ii) are made by means of a bifunctional chemical bonding agent.

7. Immunogenic product according to claim 6, **characterized in that** the said chemical bonding agent comprises at least two free aldehyde functions.

8. Immunogenic product according to claim 7, **characterized in that** the said bonding agent is glutaraldehyde.

9. Immunogenic product according to any one of claims 1 to 8, **characterized in that** the antigenic protein(s) (i) is/are chosen from among interleukine-4, alpha interferon, gamma interferon, VEGF, interleukine-10, TNF alpha, TGF beta, interleukine-5 and interleukine-6.

10. Immunogenic product according to any one of claims 1 to 9, **characterized in that** it is chosen from among the products comprising the following heterocomplexes, in which the antigenic proteins (i) on the one hand and the carrier protein molecule (ii) on the other hand, are respectively:
a) (I) IL-4 and (ii) KLH;
b) (i) alpha interferon and (ii) KLH;
c) (i) VEGF and (ii) KLH;
d) (i) IL-10 and (ii) KLH;
e) (i) alpha interferon and (ii) gp160 of HIV1
f) (i) IL-4 and (ii) the allergenic antigen Bet v 1;
g) (i) VEGF and (ii) the E7 protein of a Papilomavirus, and
h) (i) TNF alpha and (ii) KLH.

11. Composition comprising an immunogenic product according to any one of claims 1 to 10.

12. Pharmaceutical composition containing an immunogenic product according to any one of claims 1 to 10, in association with one or more physiologically compatible excipients.

13. Immunogenic composition comprising an immunogenic product according to any one of claims 1 to 10, in association with one or more physiologically compatible excipients.

14. Vaccine composition comprising an immunogenic product according to any one of claims 1 to 10, in association with one or more physiologically compatible excipients.

15. Immunogenic composition or vaccine composition according to any one of claims 13 or 14, **characterized in that** it comprises the immunoadjuvant CpG.

16. Process for the preparation of an immunogenic product according to any one of claims 1 to 9, **characterized in that** it comprises the following steps:
a) incubating the antigenic proteins (i) consisting of cytokines produced naturally by a subject, and the carrier molecule (ii) in a molar ratio (i): (ii) of 5 : 1 to 50 : 1, in the presence of a chemical bonding agent;
b) stabilizing the heterocomplexes formed at step a) by treatment with formaldehyde, and
c) recovering the immunogenic product containing immunogenic heterocomplexes which is prepared at step b).

17. Process according to claim 16, **characterized in that** the chemical bonding agent is glutaraldehyde.
